# EUROPEAN PATENT APPLICATION

(11) **EP 1 837 396 A1**
(43) Date of publication of application: **26.09.2007**
(21) Application number: 06006045.6
(22) Date of filing: 23.03.2006
(51) Int. Cl.: C12N 9/02, C12N 15/53, C12N 15/82, C12N 5/14, C07K 16/16, C12P 21/02, C07D 489/02, A61K 38/44

(54) **Salutaridine reductase and morphine biosynthesis**

(71) Applicant: Leibniz-Institut für Pflanzenbiochemie (IPB), 06120 Halle (Saale) (DE)
(72) Inventor: Ziegler, Jörg, Dr., 06112 Halle (DE); Kutchan, Toni M. Prof. Dr., St. Louis, Missouri 63141 (US)
(74) Representative: Grünecker, Kinkeldey, Stockmair & Schwanhäusser Anwaltssozietät

(57) **Abstract**

Plants of the order Ranunculales, especially members of the species *Papaver*, accumulate a large variety of benzylisoquinoline alkaloids with about 2,500 structures. But only the opium poppy, *Papaver somniferum,* and *Papaver setigerum,* are able to produce morphinan alkaloids such as the analgesic morphine or the antitussive codeine. We investigated the molecular basis for this exceptional biosynthetic capability by comparison of alkaloid profiles with gene expression profiles between sixteen different *Papaver* species and identified one cDNA which exhibits very similar expression pattern to previously isolated cDNAs coding for enzymes in benzylisoquinoline biosynthesis and which showed the highest amino acid identity to reductases in menthol biosynthesis. When expressed, the protein encoded by this cDNA reduced the keto group of salutaridine yielding salutaridinol, an intermediate in morphine biosynthesis. The stereoisomer epi-salutaridinol was not formed. The encoded protein was identified as salutaridine reductase (SalR; EC 1.1.1.248) and it was found to belongs to the family of the short chain dehydrogenases / reductases.

SEQUENCE LISTING

<110> Institut für Pflanzenbiochemie (IPB)

<120> Salutaridine Reductase and Morphine Biosynthesis

<130> EP38068FZ121pau

<160> 2

<170> PatentIn version 3.3

<210> 1
<211> 936
<212> DNA
<213> Papaver somniferum

<220>
<223> Salutaridine Reductase (Open Reading Frame)

<400> 1
atgcctgaaa catgtccaaa tactgttaca aagaggaggt gtgcagttgt tactggcgga 60

aacaagggta tcggatttga gatttgtaag caattatctt ctaatggaat catggttgtt 120

ttaacttgta gagatgtaac taaaggtcat gaagctgttg aaaaactcaa aaattccaat 180

catgagaatg tggtttttca tcaacttgat gttacggatc caattgctac tatgtcttct 240

ttagcggatt tcattaaaac acacttcgga aagcttgata tcttggtaaa caatgctggg 300

gttgcaggtt tttcagttga tgctgatcgt tttaaggcaa tgataagtga cattggagag 360

gattcagagg agctcgtgaa gatctacgaa aaaccagaag cccaagaatt aatgtcagag 420

acatatgaat tagcagaaga atgtctcaaa ataaattaca acggtgttaa atcggtaacc 480

gaagttctaa ttcctttact tcaactatct gattcaccaa gaattgttaa tgtttcatca 540

tccacgggaa gcctcaagta tgtatccaat gaaacagctc tagagatact tggagatggt 600

gatgcattaa cagaagagag aattgacatg gtagtgaata tgcttcttaa ggattttaag 660

gaaaatttga tcgaaacaaa tgggtggcct agtttcggag ctgcatacac aacatcaaaa 720

gcatgtttga atgcttacac aagggtgtta gcaaacaaaa ttcccaaatt tcaggtcaat 780

tgtgtttgtc ctgrtttggt taaaacagaa atgaactacg gcattggaaa ttatactgcc 840

gaagaaggtg ctgarcatgt agtcagaata gctcttttcc ccgacgatgg accttctggt 900

tttttctatg attgttcaga actatctgca ttttga 936

<210> 2
<211> 311
<212> PRT
<213> Papaver somniferum

<220>
<223> Salutaridine Reductase

<220>
<221> misc_feature
<222> (265)..(265)
<223> Xaa can be any naturally occurring amino acid

<400> 2

Met Pro Glu Thr Cys Pro Asn Thr Val Thr Lys Arg Arg Cys Ala Val
1               5                   10                  15

Val Thr Gly Gly Asn Lys Gly Ile Gly Phe Glu Ile Cys Lys Gln Leu
            20                  25                  30

Ser Ser Asn Gly Ile Met Val Val Leu Thr Cys Arg Asp Val Thr Lys
        35                  40                  45

Gly His Glu Ala Val Glu Lys Leu Lys Asn Ser Asn His Glu Asn Val
    50                  55                  60

Val Phe His Gln Leu Asp Val Thr Asp Pro Ile Ala Thr Met Ser Ser
65                  70                  75                  80

Leu Ala Asp Phe Ile Lys Thr His Phe Gly Lys Leu Asp Ile Leu Val
                85                  90                  95

Asn Asn Ala Gly Val Ala Gly Phe Ser Val Asp Ala Asp Arg Phe Lys
            100                 105                 110

Ala Met Ile Ser Asp Ile Gly Glu Asp Ser Glu Glu Leu Val Lys Ile
        115                 120                 125

Tyr Glu Lys Pro Glu Ala Gln Glu Leu Met Ser Glu Thr Tyr Glu Leu
    130                 135                 140

Ala Glu Glu Cys Leu Lys Ile Asn Tyr Asn Gly Val Lys Ser Val Thr
145                 150                 155                 160

Glu Val Leu Ile Pro Leu Leu Gln Leu Ser Asp Ser Pro Arg Ile Val
                165                 170                 175

Asn Val Ser Ser Ser Thr Gly Ser Leu Lys Tyr Val Ser Asn Glu Thr
            180                 185                 190

Ala Leu Glu Ile Leu Gly Asp Gly Asp Ala Leu Thr Glu Glu Arg Ile
        195                 200                 205

Asp Met Val Val Asn Met Leu Leu Lys Asp Phe Lys Glu Asn Leu Ile
    210                 215                 220

Glu Thr Asn Gly Trp Pro Ser Phe Gly Ala Ala Tyr Thr Thr Ser Lys
225                 230                 235                 240

Ala Cys Leu Asn Ala Tyr Thr Arg Val Leu Ala Asn Lys Ile Pro Lys
                245                 250                 255

Phe Gln Val Asn Cys Val Cys Pro Xaa Leu Val Lys Thr Glu Met Asn
            260                 265                 270

Tyr Gly Ile Gly Asn Tyr Thr Ala Glu Glu Gly Ala Glu His Val Val

        275                 280                 285

Arg Ile Ala Leu Phe Pro Asp Asp Gly Pro Ser Gly Phe Phe Tyr Asp
    290                 295                 300

Cys Ser Glu Leu Ser Ala Phe
305                 310

## Description

### FIELD OF THE INVENTION

The present application relates to proteins having salutaridine reductase activity and variants and derivatives thereof. The present invention also relates to nucleic acid molecules encoding said proteins, and variants and derivatives thereof. The present invention further relates to transgenic plants, plant part, or plant cells having altered alkaloid profiles and the use thereof. In addition, the present invention relates to the production of morphinan alkaloids.

### BACKGROUND OF THE INVENTION

The benzylisoquinoline alkaloids comprise a group of secondary metabolites with roughly 2,500 structures. Some of them possess important pharmacological effects such as the vasodilator papaverine, the antimicrobial sanguinarine, the antitussive codeine or the analgesic morphine (Kutchan, 1998; Kutchan et al., 2004).

The biosynthesis of the benzylisoquinolines starts with the condensation of the tyrosine derived tyramine and p-hydroxyphenylacetaldehyde by norcoclaurine synthase (NCS), which represents the first benzylisoquinoline specific enzyme of the pathway. The product (*S*)-norcoclaurine is further converted by several S-adenosylmethionine-dependent methyltransferases and one P450-monoxygenase. The (*S*)-norcoclaurine 6-0-methyltransferase (6-OMT) methylates the 6-hydroxy position of (*S*)-norcoclaurine to (S)-coclaurine, which then undergoes N-methylation by coclaurine *N*-methyltransferase (CNMT) to yield N-methylcoclaurine. The P450 dependent monooxygenase *N*-methylcoclaurine 3'-hydroxylase (Cyp80B3) inserts a hydroxyl group at the 3*'*-position and the resulting 3'-hydroxy-N-methylcoclaurine is methylated by 3'-hydroxy-N-methylcocaurine 4'-*O-*methyltransferase (4'-OMT) to (S)-reticuline (Kutchan, 1998; Kutchan et al., 2004). Up to this step, the biosynthesis is the same for all benzylisoquinoline structures.

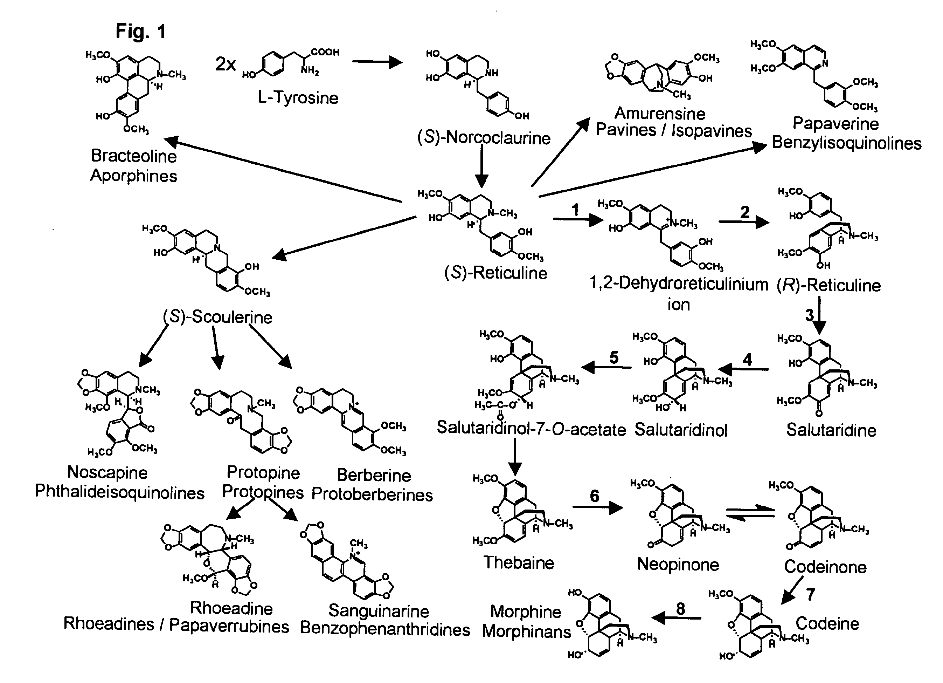

The highly structural variety of the benzylisoquinoline originates from the different modifications of the central intermediate (S)-reticuline (Figure 1). This core structure can be modified by oxidations and further methylations to the true benzylisoquinolines such as papaverine. Another initial modification, leading to most of the benzylisoquinoline structures is the formation of C-C bonds between various carbon atoms. Thus, the classes of aporphine and pavine / isopavines are formed. Alternatively, (S)-scoulerine is formed, which gives further rise to numerous structural classes of benzylisoquinolines such as the phthalideisoquinolines, the protoberberines or the protopines (Kutchan, 1998). The latter class can be further modified to the benzylisoquinoline classes of the papaverrubines / rhoeadines and the benzophenanthridines (Rönsch, 1986; Kutchan, 1998). Reactions after the initial C-C-coupling include *N*- and *O*-methylations, reduction of double bonds, desaturation and insertions of oxygen atoms or formation of methylenedioxy bridges, presumably by P450-monoxygenases (Kutchan, 1998).

Whereas all of these classes of benzylisoquinolines are formed by modification of (S)-reticuline, the pathway to the morphinans is initiated by the conversion of stereochemistry at C-1 from (S)- to (R)-reticuline, catalyzed in a two step reaction including an oxidation of (S)-reticuline by reticuline oxidase to 1,2,-dehydroreticulinium ion and subsequent reduction of the ion to (R)-reticuline by 1,2,-dehydroreticulinium ion reductase (Figure 1). In analogy to the other benzylisoquinoline classes, a carbon-carbon phenol coupling between C-12 and C-13 is formed by the P450 oxygenase salutaridine synthase to form salutaridine. A stereospecific, NADPH dependent salutaridinee reductase reduces the keto group at C-7 and 7(S)-salutaridinot is formed, which is further modified at the same position by acetylation catalyzed by 7(S)-salutaridinol-O-acetyltransferase (SaIAT). Elimination of the acetyl residue through formation of an oxide bridge yields thebaine, the first pentacyclic alkaloid of the morphinan type. Two demethylations and the reduction of codeinone to codeine by codeinone reductase (COR) result in the formation of morphine (Kutchan, 1998; Kutchan et al., 2004).

The basic benzylisoquinoline pathway up to (S)-reticuline has been elucidated on the enzyme and the molecular biology level during the past years and for all enzymes cDNAs have been isolated from several plant species (Facchini and DeLuca, 1994; Frick and Kutchan, 1999; Huang and Kutchan, 2000; Morishige et al., 2000; Choi et al., 2002; Facchini and Park, 2003; Ounaroon et al., 2003; Samanani et al., 2004; Ziegler et al. 2005). However, much less is known about the pathways downstream of (S)-reticuline.

In some cases, such as for the pavines /isopavines, the precise metabolic pathways are not yet known. For the biosynthesis of the other benzylisoquinolines classes, the metabolic flow has been elucidated and some enzymes have been measured and partially purified, as in the case of the protoberberines and the benzophenanthridines (Kutchan, 1998). In this pathway, some cDNAs could be isolated, such as the berberine bridge enzyme, scoulerine 9-*O*-methyltransferase, columbamine-*O*-methyltransferase, canadine synthase and (*R*,*S*)-reticuline 7-*O*-methyltransferase (Dittrich and Kutchan, 1991; Takeshita et al., 1995; Morishige et al., 2002; Ikezawa et al., 2003; Ounaroon et al., 2003).

Because of its high economical importance, the morphinan branch of the benzylisoquinoline pathway has been under intensive investigations in the past years and two enzymes, SalAT and COR, have been purified to homogeneity and the encoding cDNAs subsequently cloned (Lenz and Zenk, 1995a; Grothe et al., 2001; Lenz and Zenk, 1995b; Unterlinner et al., 1999). With the exception of the demethylation steps downstream of thebaine, all the other enzymes of the pathway have been characterized and partially purified (De-Eknamkul and Zenk, 1992; Gerardy and Zenk, 1992; Gerardy and Zenk, 1993).

There are three reductive steps in the biosynthesis of morphine, the conversion of 1,2-dehydroreticulinium ion to (R)-reticuline, and the reductions of the keto group of salutaridine and codeinone to salutaridinol and codeine, respectively. The latter, COR has been cloned from *P. somniferum* and had been shown to be a member of the aldo-keto reductase family (Unterlinner et al., 1999). 1,2-dehydroreticulinium ion reductase and salutaridinee reductase have been partially purified and proven to be NADPH dependent, but to which class of reductases these enzymes belong is not known (De-Eknamkul and Zenk, 1992; Gerardy and Zenk, 1993).

Apart from the aldo-keto reductases, which catalyze reactions in plant secondary metabolism (Welle et al., 1991), the large family of short-chain dehydrogenases / reductases (SDR) have been also shown to participate in the biosynthesis of different secondary metabolites. SDRs have been shown to be involved in the stereospecific reduction of (-)-menthone to either (-)-menthol or (+)-neomenthol, respectively, in monoterpene biosynthesis of peppermint (Davis et al., 2005). In the biosynthesis of tropane alkaloids two stereospecific tropinone reductases catalyzing the reduction of tropinone to tropine and pseudo-tropine have been isolated from *Hyoscyamus niger* (Nakajima et al., 1999).

Benzylisoquinolines are mainly produced by plants belonging to the order of the Ranunculales. Especially plants of the genus *Papaver,* of which 70 species are described, accumulate a rich spectrum of different benzylisoquinolines (Preininger, 1986).

The pharmacologically most important species is the opium poppy *Papaver somniferum.* Together with *P. bracteatum* and *P. setigerum,* this is the only plant species known to date capable of pentacyclic morphinan alkaloid biosynthesis. Whereas *P. bracteatum* accumulates thebaine as the end product, only *P. setigerum* and *P. somniferum* are able to further metabolize this molecule to codeine and morphine.

The latter plant has been subjected to extensive breeding programs, partially in order to change the alkaloid profile and, mainly, to massively increase the content of specific alkaloids. Although these breeding programs succeeded in the generation of high-alkaloid producing opium poppy varieties, the molecular mechanisms causing these phenotypes are not known. Likewise, the reason why among all these different, but genetically closely related *Papaver* species, only *P. bracteatum, P. somniferum* and *P. setigerum* are capable of the production of pentacyclic morphinan alkaloids is not known.

Therefore, there is a need in the field to identify the differences between *Papaver* species that are capable of producing morphinan alkaloids and those that are not. Furthermore, there is a need in the field to identify additional enzymes that are involved in the biosynthesis of morphinan alkaloids.

One method by which to investigate the underlying mechanisms making the opium poppy so distinct from the other *Papaver* species is the global analysis of gene expression by array technologies. Recording the expression profiles of many cDNAs in various *Papaver* species and correlating them with the benzylisoquinoline profile could lead to the discovery of cDNAs responsible for the unique quantitative and qualitative chemotype of *P. somniferum.* Using this approach, the comparison between morphine containing and morphine-free *Papaver* species showed a higher expression of genes involved in benzylisoquinoline biosynthesis in *P. somniferum* and resulted in the isolation and characterization of a cDNA coding for 4'-OMT (Ziegler et al., 2005). In this invention, we include additional *Papaver* species and expressed sequence tags in the comparisons and could identify a cDNA encoding a member of the short chain dehydrogenase / reductase familiy exhibiting significantly higher expression in *P. somniferum.* Characterization of the recombinant protein identified it as salutaridine reductase of morphine biosynthesis.

### SUMMARY OF THE INVENTION

The present invention provides a nucleic acid encoding a polypeptide having salutaridine reductase activity.

The nucleic acid of the present invention comprises a nucleotide sequence selected from the group consisting of:
(a) the nucleotide sequence as set forth in SEQ ID NO:1;
(b) a nucleotide sequence encoding the polypeptide as set forth in SEQ ID NO:2;
(c) a nucleotide sequence encoding a polypeptide having the amino acid sequence as set forth in SEQ ID NO:2 further comprising an amino-terminal methionine;
(d) a nucleotide sequence that is at least 70% identical to the nucleotide sequence of any of (a)-(c) over its entire length, wherein the nucleotide sequence encodes a polypeptide having salutaridine reductase activity;
(e) a nucleotide sequence encoding a polypeptide that is at least 70% identical to the polypeptide as set forth in SEQ ID NO:2 over its entire length, wherein the encoded polypeptide has salutaridine reductase activity;
(f) a nucleotide sequence which hybridizes under highly stringent or moderately stringent conditions to the nucleotide sequence as set forth in SEQ ID NO:1, wherein the nucleotide sequence encodes a polypeptide having salutaridine reductase activity;
(g) a nucleotide sequence that is an ortholog of the nucleotide sequence as set forth in SEQ ID NO:1, wherein the nucleotide sequence encodes polypeptide having salutaridine reductase activity;
(h) a nucleotide sequence that is an allelic variant or a splice variant of the nucleotide sequence as set forth in SEQ ID NO:1, wherein the nucleotide sequence encodes a polypeptide having salutaridine reductase activity;
(i) a fragment of the nucleotide sequence of any of (a)-(h) encoding a polypeptide of at least 25 amino acid residues, wherein the encoded polypeptide has salutaridine reductase activity;
(j) a nucleotide sequence encoding a polypeptide as set forth in SEQ ID NO: 2 with at least one amino acid substitution, wherein the encoded polypeptide has salutaridine reductase activity;
(k) a nucleotide sequence encoding a polypeptide as set forth in SEQ ID NO:2 with at least one amino acid insertion, wherein the encoded polypeptide has salutaridine reductase activity;
(l) a nucleotide sequence encoding a polypeptide as set forth in SEQ ID NO:2 with at least one amino acid deletion, wherein the encoded polypeptide has salutaridine reductase activity;
(m) a nucleotide sequence encoding a polypeptide as set forth in SEQ ID NO:2 which has a carboxyl- and/or amino-terminal truncation, wherein the encoded polypeptide has salutaridine reductase activity;
(n) a nucleotide sequence encoding a polypeptide as set forth in SEQ ID NO:2 with at least one modification selected from the group consisting of amino acid substitution, amino acid insertion, amino acid deletion, carboxyl-terminal truncation, and amino-terminal truncation, wherein the encoded polypeptide has salutaridine reductase activity; and
(o) a nucleotide sequence complementary to any of (a)-(n), wherein the nucleotide sequence is at least 15 nucleotides in length.

The present invention also provides an expression vector comprising the nucleic acid molecule of the present invention, operably linked to transcription regulatory sequences.

In one embodiment, the transcription regulatory sequences are heterologous transcription regulatory sequences which are different from the native transcription regulatory sequences for salutaridine reductase.

The present invention further provides a host cell comprising the expression vector of the present invention.

The host cell of the present invention may be selected from a prokaryotic cell and a eukaryotic cell, preferably a bacterial cells, a yeast cell, a mammalian cells, and a plant cell.

The present invention provides a transgenic plant comprising the nucleic acid or the vector of the present invention, preferably stably integrated into the plant genome.

In one embodiment, the transgenic plant belongs to the genus *Papaver,* perfereably to a species selected from the group consisting of *Papaver somniferum, Papaver bracteatum, Papaver cylindricum, Papaver orientale, Papaver setigerum, Papaver pseudo-orientale, Papaver lauricola, Papaver persicum, Papaver caucasium, Papaver carmeli, Papaver arenarium, Papaver argemone, Papaver atlanticum, Papaver commutatum, Papaver dubium, Papaver fugax, Papaver hybridum, Papaver lateritium, Papaver oreophilum, Papaver pavonium, Papaver pilosum, Papaver pyrenaicum.*

The present invention also provides parts of the transgenic plant of the present invention, such as seed, seed pod, straw or root.

The present invention provides a polypeptide having salutaridine reductase activity. The polypeptide of the present invention is encoded by a nucleic acid of any of (a)-(n) of the present invention.

The present invention also provides a fusion polypeptide comprising the polypeptide of the present invention fused to a heterologous amino acid sequence.

The present invention further provides an antibody or a fragment thereof that specifically binds to the polypeptide of the present invention.

In a preferred embodiment, the antibody or the fragment thereof is a monoclonal antibody or a fragment thereof.

The present invention in addition provides a hybridoma that produces the monoclonal antibody of the present invention.

The present invention provides a method for detecting and/or quantitating the amount of the polypeptide of the present invention, comprising the steps of:
(a) contacting a sample with the antibody or a fragment thereof of the present invention;
(b) detecting the complex formed between the polypeptide and the antibody or a fragment thereof; and
(c) optionally quantitating the amount of the complex.

The present invention also provides a process for producing the polypeptide of the present invention comprising culturing the host cell of the present invention under suitable conditions to express the polypeptide, and optionally isolating the polypeptide from the culture, wherein the expression vector contained in the host cell comprises a nucleic acid of any of (a)-(n) of the present invention.

The present invention provides a method for producing a plant or a plant part having altered alkaloid production, comprising the steps of:
(a) introducing the nucleic acid of the present invention into a cell or a part of an alkaloid-producing plant;
(b) growing the product of (a) under suitable conditions which permit the expression of the nucleic acid; and
(c) optionally regenerating a whole plant from said cell or part.

The present invention also provides a method for producing a plant cell culture having altered alkaloid production, comprising the steps of:
(a) introducing the nucleic acid of the present invention into a cell of an alkaloid-producing plant;
(b) growing the product of (a) under suitable conditions which permit the expression of the nucleic acid; and
(c) establishing a cell culture from said cell.

In one embodiment, the plant belongs to the genus *Papaver,* preferably to a species selected from the group consisting of *Papaver somniferum, Papaver bracteatum, Papaver cylindricum, Papaver orientale, Papaver setigerum, Papaver pseudo-orientale, Papaver lauricola, Papaver persicum, Papaver caucasium, Papaver carmeli, Papaver arenarium, Papaver argemone, Papaver atlanticum, Papaver commutatum, Papaver dubium, Papaver fugax, Papaver hybridum, Papaver lateritium, Papaver oreophilum, Papaver pavonium, Papaver pilosum, Papaver pyrenaicum.*

The present invention provides a method for producing morphinan alkaloids, comprising the steps of:
(a) growing the plant obtainable by the method for producing a plant or a plant part having altered alkaloid production under suitable conditions which permit the expression of the nucleic acid of any of (a)-(n), wherein the plant comprises the nucleic acid of any of (a)-(n) of the present invention; and
(b) recovering morphinan alkaloids from the plant or the plant part.

The present invention provides an alternative method of producing morphinan alkaloids, comprising the steps of:
(a) culturing the plant cell culture obtainable by the method for producing a plant cell culture having altered alkaloid production under suitable conditions which permit the expression of the nucleic acid of any of (a)-(n), wherein the plant cell comprises the nucleic acid of any of (a)-(n) of the present invention; and
(b) recovering morphinan alkaloids from the plant cell culture.

The present invention provides a further alternative method of producing morphinan alkaloids, comprising the steps of:
(a) contacting a substrate-containing sample with the polypeptide of the present invention under suitable conditions which allow the reduction of salutaridine into salutaridinol; and
(b) recovering morphinan alkaloids.

In one embodiment, the morphinan alkaloid is salutaridinol.

The present invention provides the use of the nucleic acid or the vector of the present invention for modulating alkaloid production in a plant, a plant part, or a plant cell.

The present invention also provides the use of the host cell, the transgenic plant or a part thereof, or the polypeptide of the present invention for producing morphinan alkaloids.

The present invention further provides the use of the transgenic plant or a part thereof of the present invention for the preparation of a composition for reducing and/or eliminating pain and/or reducing and/or suppressing coughing.

### BRIEF DESCRIPTION OF THE DRAWINGS

**Figure 1.** Benzylisoquinoline Pathway. One representative compound for each benzylisoquinoline class with emphasis on morphine biosynthesis is shown. The enzymes indicated are: 1, (R)-reticuline oxidase; 2, 1,2-dehydroreticulinium ion reductase; 3, salutaridine synthase; 4, salutaridine reductase; 5, salutaridinol 7-*O*-acetyltransferase; 6+8, demethylations of unknown enzymatic mechanism; 7, codeinone reductase.

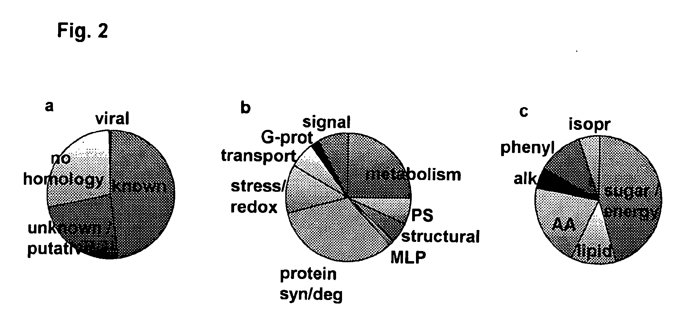

**Figure 2.** Classification and Expression Analyses of ESTs. a: for all sequences: no homology = sequences with homology of an e-value >10⁻⁹; unknown / putative = sequences with homology to uncharacterised expressed proteins or hypothetical proteins; b: for sequences showing homology to a database entry: PS = photosynthesis; structural = cytoskeleton, cell wall synthesis / degradation proteins; MLP = major latex proteins; protein syn/deg = cell growth, cell division, DNA- and protein synthesis; stress / redox = abiotic / biotic stress / redox control; G-prot = G-proteins; signal = cellular communication / signal transduction. c: for sequences coding for proteins involved in metabolism: AA = amino acid metabolism; alk = alkaloid biosynthesis; phenyl = phenylpropanoid biosynthesis; isopr = isoprenoid biosynthesis.

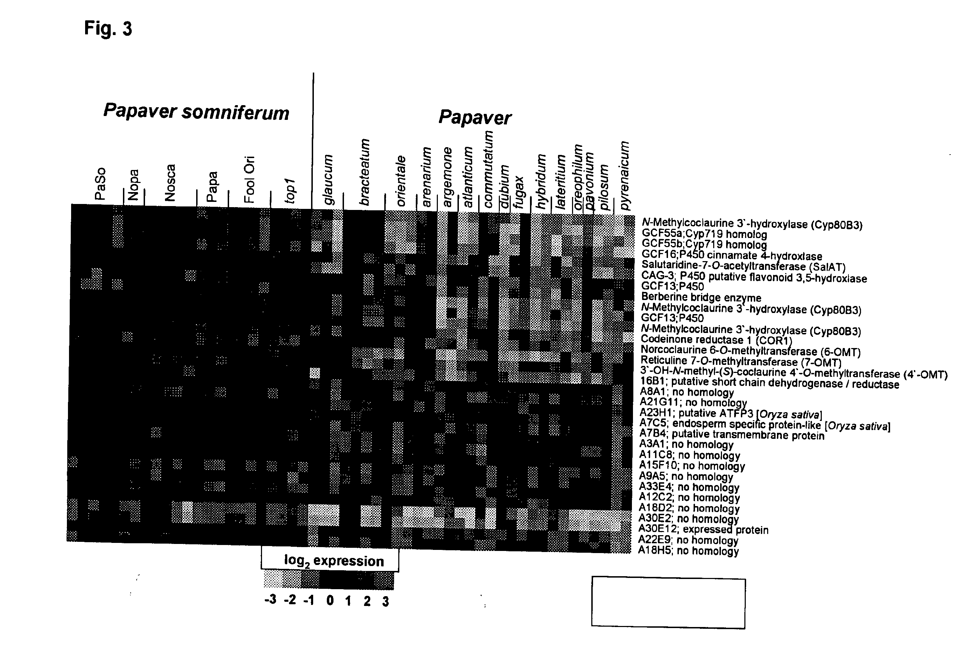

**Figure 3.** Clustered Display of Gene Expression Analysis of *Papaver* Species. One cluster showing increased expression in *P. somniferum* varieties is shown. Indicated in blue: cDNAs coding for enzymes in benzylisoquinoline biosynthesis; red: cDNA characterized in this study. The *P. somniferum top1* mutant is described in Millgate et al., 2004.

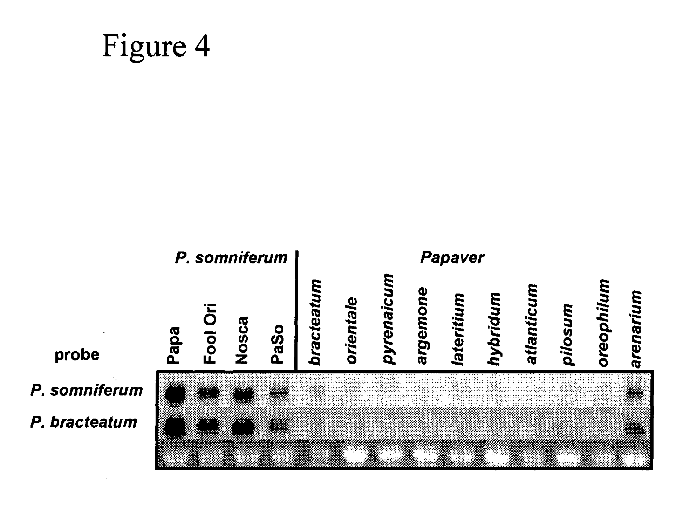

**Figure 4.** RNA Gel Blot Analysis. Total RNA from different *Papaver* species was separated by RNA gel electrophoresis, blotted on a membrane and hybridized with [□̃ ³²P]dATP labelled 16B1 probe from *P. somniferum* (top) or *P. bracteatum* (bottom). As RNA loading control, the photographs of ethidium bromide visualized RNA are shown.

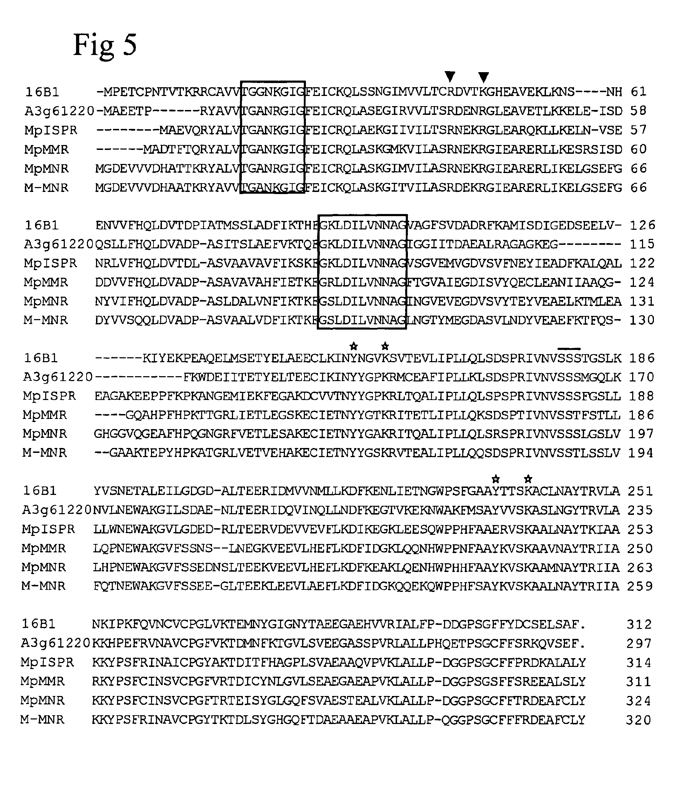

**Figure 5.** Protein Sequence Alignment of Short Chain Dehydrogenases
   The amino acid sequence alignments were performed using the ClustalW application (Thompson et al., 1994) of MegAlign (DNASTAR Inc).
   The protein sequence of 16B1 isolated from *P. somniferum* was aligned to the sequences of an SDR from *Arabidopsis thaliana* (At3g61220) of unknown function, to *Mentha x piperita* (- )isopiperitenone reductase (MpISPR; GenBank accession number AY300162), (-)-menthone:(-)-menthol reductase (MpMMR; AY288138), (-)-menthol:(+)-neomenthol reductase (MpMNR; AY288137) and to the SDR from peppermint isolated in this study with (-)-menthol:(+)-neomenthol reductase activity (M-MNR). The nucleotide binding motif TGxxxGhG and the motif GxhDhhhNNAG stabilizing the central β-sheet are boxed. Asterisks indicate the possible YxxxK catalytic centers and possible catalytic Ser-residues are emphasised by a line on top of the sequence. Residues responsible for the preference for NADP(H) over NAD(H) are indicated by arrowheads.
**Figure 6.** HPLC Analysis of Salutaridine Reductase Assays. **(A)** salutaridine and salutaridinol standards; **(B-D)** assays with extracts from bacteria bearing **(B)** the overexpression vector pQE30 without insert and induced with 1 mM IPTG, **(C)** the overexpression vector pQE30 with insert 16B1 without IPTG induction, and **(D) ;** the overexpression vector pQE30 with insert and induced with 1 mM IPTG.
**Figure 7.** Chemical Structures of Substrates Tested for Salutaridine Reductase Specificity. The substrates and the expected reduced products are shown.
**Figure 8.** Neighbour-Joining Phylogenetic Tree of Selected Plant Proteins Catalyzing Reductions in Plant Secondary Metabolism.
   The amino acid sequence alignments were performed using the ClustalW application (Thompson et al., 1994) of MegAlign (DNASTAR Inc), and the tree was generated and visualized with the Treecon (Yves van de Peer, University of Konstanz, Germany) software. Bootstrap values in percent of 1,000 trials are indicated. The source and the GenBank accession numbers are: At3g61220 SDR from *Arabidopsis thaliana* with low (-)-menthone:(+)-neomenthol reductase activity, accession NM115986; PsSaIR (salutaridine reductase) from *Papaver somniferum,* DQ31621; MpISPR ((-)-isopiperitenone reductase), AY300162; MpMNR ((-)-menthone:(+)-neomenthol reductase), AAQ55959; MpMMR ((-)-menthone:(-)-menthol reductase), AAQ55960; MpPuIR ((+)-pulegone reductase), AY300163; MpISPD ((-)-isopiperitenol dehydrogenase), AY641428; all from *Mentha* x *piperita;* HnTR-I+HnTRII (tropinone reductase I and II) from *Hyoscyamus niger,* D88156 and L20485; SDH_Fi321 (secoisolariciresinol dehydrogenase) from *Forsythia x intermedia,* AAK38665; PsCOR1 (codeinone reducatse 1) from *Papaver somniferum,* AF108432; GmCHR (chalcone reductase) from *Glycine max,* X55730.

### DETAILED DESCRIPTION OF THE INVENTION

### Correlation of Alkaloid Profiles and Gene Expression Profiles between Papaver Species Detects cDNAs of Benzylisoquinoline Biosynthesis in P. somniferum

The aim of the present study was the isolation and characterization of new cDNAs that might be implicated in benzylisoquinoline accumulation in *Papaver* species. Most of the cDNAs coding for enzymes involved in benzylisoquinoline biosynthesis have been obtained by two approaches. The first one, a classical and very targeted approach, started with the purification of the protein from the plant followed by amino acid sequencing and cDNA cloning using degenerate primers (Unterlinner etal., 1999; Grothe et al., 2001). The second one relied on the sequence homology between members of one enzyme family, such as methyltransferases or P450 monooxygenases (Frick and Kutchan, 1999; Huang and Kutchan, 2000). Using primers directed against conserved protein domains, a number of cDNAs coding for the examined protein family were isolated and each recombinant protein was subsequently tested for its capability to catalyze the desired reaction.

Here, a broader approach was used to isolate new cDNAs. An EST-library prepared from *P. somniferum* RNA was sequenced in order to collect a large amount of sequence information on cDNA clones. Since most of the cDNAs cannot be classified with respect to their function in benzylisoquinoline biosynthesis simply by sequence homology, the expression profile was correlated to the alkaloid profile in order to obtain hints about possible biological functions of individual cDNAs. Successes of this method have been obtained in the characterization of terpene synthases, methyl ketone synthase, cytochrome P450 monooxygenases, and methyltransferases, where the involvement in specific biosynthetic steps could be elaborated by comparison of the expression profile with the metabolite profile (Gang et al., 2002; Guterman et al., 2002; Lavid et al., 2002; Chen et al., 2003; Aharoni et al., 2004; Iijima et al., 2004; Fridman et al., 2005, Fridman and Pichersky, 2005, Ziegler et al., 2005).

In these investigations, the authors used one variety or wild type accumulating low amounts of certain metabolites and compared it with another cultivar of the same plant species producing large amounts of these metabolites. The use of different cultivars of the same plant species has the advantage that differences in gene expression due to morphological characteristics are diminished, and, therefore, the probability of isolating the desired cDNAs is increased.

This is in contrast to the approach presented in this paper, where we compared different *Papaver* species, which exhibit in some cases very different morphology. Comparing only two species could, therefore, result in many genes that are differentially expressed and only a very minor portion of these genes may be responsible for the different alkaloid profile of the two species.

This is exemplified by the comparison of *P. somniferum* varieties with *P. pyrenaicum,* where 225 cDNAs are differentially expressed, among them typical cDNAs involved in the establishment of cell differentiation such as cell division control proteins. The comparison of *P. somniferum* with *P. glaucum,* which show rather similar morphological characteristics yielded 133 differentially expressed genes, which, obviously, will also not all be responsible for the capability of *P. somniferum* to produce morphinan alkaloids. Combination of both datasets, however, yielded 93 differentially expressed genes. Irrespective of the different habitats of the plants, these 93 cDNAs have in common that their differential expression coincides with the occurrence of morphinans in *P. somniferum.*

Thus, by inclusion of several more *Papaver* species showing alkaloid profiles differing from alkaloid profile unique to *P. somniferum,* the number of cDNAs could be reduced to 3% of all investigated cDNAs. Although many of them will probably be responsible for processes other than benzylisoquinoline biosynthesis, it is notable that none of these cDNAs showed homology to proteins involved in general cellular functions.

It is also striking that all cDNAs coding for benzylisoquinoline biosynthesis thus far identified are located in one cluster showing higher expression in *P. somniferum* (i.e. *OMTs, SalAT* and *COR,* Figure 3). Whereas the increased expression of the morphinan-specific cDNAs *SalAT* and *COR* can be ascribed to morphinan accumulation, the higher expression of the enzymes upstream of the central intermediate (*S*)-reticuline, such as 6-OMT, 4'-OMT and Cyp80B3 might be attributed to the overall higher amounts of alkaloids in *P. somniferum* compared to the other *Papaver* species.

Nevertheless, the presence of these cDNAs in clusters with increased expression in *P. somniferum* shows that by comparison of alkaloid profiles with gene expression profiles between distinct species, cDNAs involved in the biosynthesis of specific alkaloids can be detected, irrespective of morphological differences. Since cDNAs involved in benzylisoquinoline biosynthesis tightly cluster in this analysis, it is assumed that other cDNAs of unkown function present in that cluster could also have some function in benzylisoquinoline biosynthesis.

### Clone 16B1 Showing Increased Expression in Morphinan Alkaloid-Containing Papaver Species Encodes Salutaridine Reductase

One of the cDNAs with increased expression in *P. somniferum* showed the highest amino acid similarity to the protein family of short chain dehydrogenases / reductases. These enzymes have been shown to act in several secondary product biosynthetic pathways, such as in terpene or tropane alkaloid metabolism (Davis et al., 2005; Ringer et al., 2005; Nakajima et al., 1999). According to the expression profile of this cDNA showing increased expression in morphinan alkaloid-producing plants, it was assumed that the enzyme might catalyse either the reduction of 1,2-dehydroreticulinium ion to (R)-reticuline or of salutaridine to salutaridinol.

Incubation of the recombinant enzyme with salutaridine as substrate and subsequent identification of the product by LC-MS-MS (liquid chromatography coupled to mass spectrometry - mass spectrometry) clearly showed that the enzyme catalyses the reduction of salutaridine to salutaridinol. Only the morphine precursor salutaridinol, not its stereoisomer 7-epi-salutaridinol, was formed, showing the high product specificity of the enzyme. Furthermore, other, structurally similar benzylisoquinolines were not accepted as substrates suggesting that the enzyme is specific for salutaridine.

An enzyme reducing salutaridine to salutaridinol has been previously partially purified and
characterized from *P. somniferum* (Gerardy and Zenk, 1993). The recombinant enzyme characterized in this study exhibits the same temperature optimum and pH-optimum as the protein purified from the plant. Furthermore, the *K*ₘ values for the substrate salutaridine and the cofactor NADPH are similar. Both enzymes are able to catalyse the reverse reaction from salutaridinol to salutaridine equally well, with the same pH-optima at basic conditions and with similar affinities for salutaridinol. Only the *K*ₘ value for the cofactor NADP is about 4 times higher for the recombinant protein compared to the purified native protein from *P. somniferum.* The size of the protein from *P. somniferum* was 51 kD as judged from the elution on size exclusion chromatography columns (Gerardy and Zenk, 1993). Based on the amino acid sequence, the recombinant protein has a molecular weight of 34.1 kD. However, on size exclusion chromatography, the recombinant protein elutes as a protein of 50 kD, which is in accordance to the purified native protein. The discrepancy between the calculated and the measured molecular weight for the recombinant protein is most likely attributable to the shape of the protein.

The substrate as well as the product specificity of the recombinant protein and the corroboration with the previously purified native protein from *P. somniferum* strongly suggests that the cDNA 16B1 codes for the enzyme salutaridine reductase (EC 1.1.1.248.). This is further confirmed by its higher expression in plants that are able to accumulate benzylisoquinolines of the morphinan type.

The stereospecific reduction of the keto-group at position 7 of salutaridine to salutaridinol is the prerequisite for acetylation by SalAT, which does not accept 7-*epi*-salutaridinol as substrate (Lenz and Zenk, 1995a). The subsequent elimination of the acetyl group concomitant with oxide bridge formation leads to thebaine, the first compound in the biosynthesis of morphine exhibiting the pentacyclic ring system. Thus, the high product stereospecificity of the recombinant enzyme fortifies the identification of the cDNA 16B1 as salutaridine reductase (SaIR) from *P. somniferum* as part of the biosynthesis of morphinan alkaloids.

### Salutaridine Reductase belongs to the Reductases of the Short Chain Dehydrogenase / Reductase Family

Together with *Sa*/*AT* and COR, the cDNA from a third enzyme specific for morphine biosynthesis has been isolated. In contrast to the previously cloned COR, performing a similar reaction by reducing a keto group in an NADPH dependent manner, and which belongs to the familiy of the aldo-keto reductases, SalR belongs to the SDR-family of reductases. This is indicated by the TGxxxGhG (h for hydrophobic residues) motif starting at position 18, which has a structural role in coenzyme binding, the GxhDhhhNNAG motif at position 90 possessing a structural role in stabilising the central β-sheet, and the YxxxK motif, which is essential for the catalysis as part of the proton ion transfer from the cofactor to the substrate (Jörnvall et al., 1995, Filling et al., 2002, Persson et al., 2003). Two of these motifs are present in the sequence, at position 153 and 236 (Figure 5). The presence of Ser residues at position 179, however, makes the YxxxK motif at position 236 more likely, since it has been shown that a Ser residue upstream of the YxxxK motif is necessary for catalytic activity (Filling et al., 2002). The preference of SalR for NADP(H) over NAD(H) can also be deduced from the amino acid sequence by the presence of the basic residues R or K downstream of the TGxxxGhG motif at position 44 or 48, respectively, instead of acidic residues (Persson et al., 2003). Based on these amino acid motifs, the SalR belongs to the classical SDR and can be grouped into the family cP2 according to Persson et al. (2003).

Whereas the classical SDRs are around 250 amino acids in length, the open reading frame of SalR codes for 311 amino acids. Additionally, functional units of classical SDRs are either homotetramers or homodimers, such as the tropinone reductases (Nakajima et al., 1993). As estimated by gel permeation chromatography, SalR is active as a monomeric protein. A monomeric structure has also been shown for SDRs in terpene metabolism, such as ISPR, MNR and MMR, which exhibit the highest amino acid sequence similarity to SalR of all hitherto characterized SDRs (Ringer et al. 2003; Davis et al., 2005). The monomeric structure is ascribed to a roughly 40 residue insertion preceding the catalytic Tyr forming an all helix subdomain that blocks the dimerization, as shown for porcine testicular carbonyl reductase (Ghosh et al., 2001). This amino acid stretch is also responsible for the increased size over the classical multimeric SDRs.

### Relationship of SalR to Other Reductases in Plant Secondary Metabolism

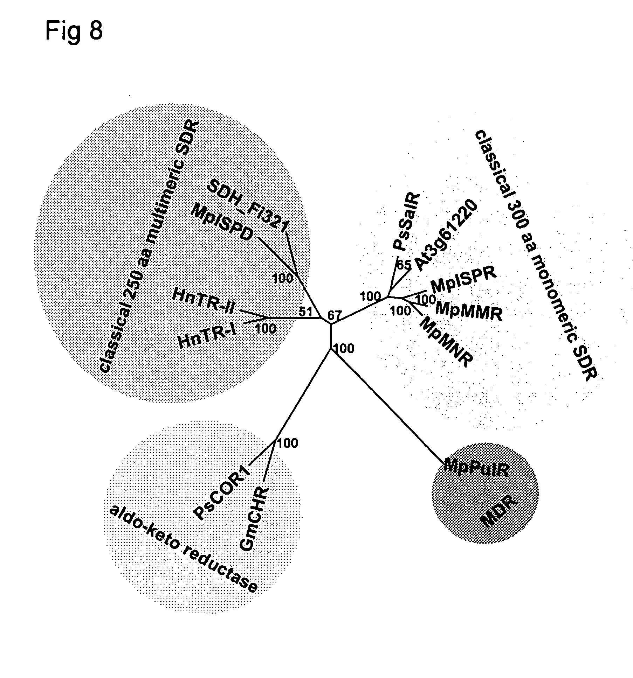

Phylogenetic analysis reveals that SalR forms one cluster together with monomeric SDRs, such as ISPR, which reduces the ring double bond in menthol biosynthesis, as well as MNR and MMR, which reduce the keto group of (-)-menthone to (+)-neomenthol or (-)-menthol, respectively (Figure 8). SaIR does not accept (-)-menthone as substrate. Conversely, the MNR cloned in this study, showing 75% identity to the MNR cloned from *Mentha x piperita,* does not reduce salutaridine, showing the high substrate specificity of these enzymes.

Interestingly, there are two additional enzymes in menthol biosynthesis catalysing reductions or dehydrogenations with low sequence similarity to this cluster. (+)-Pulegone reductase, reducing the double bond of the side chain, belongs to the family of the medium chain dehydrogenases / reductases (Ringer et al., 2003), whereas ISPD, catalysing the dehydrogenation from (-)-*trans*-isopiperitenol to (-)-isopiperitenone, is classified into the classical SDRs with a homodimeric functional unit and a subunit size of about 250 amino acids (Ringer et al., 2005). The latter enzyme, therefore, resembles tropinone reductases and secoisolariciresinol dehydrogenase, an SDR in podophyllotoxin biosynthesis (Xia et al., 2001). These enzymes form a cluster independent from the monomeric SDRs ISPR, MMR, MNR and SaIR. The latter two enzymes are not able to convert tropinone to tropine.

Furthermore, SaIR does not catalyse a similar reaction in morphine biosynthesis, the reduction of codeinone to codeine. This step is performed by COR, which belongs to the family of the aldo-keto reductases and is more related to chalcone reductase, an enzyme of flavonoid biosynthesis (Unterlinner et al., 1999, Bomati et al., 2005). Conversely, COR does not accept salutaridine as substrate (Unterlinner et al., 1999). Taken together, it is evident that reductions or dehydrogenations in plant secondary metabolism can be performed by enzymes of high substrate specificity that can be classified into several families. This is not surprising, taking into account the diversity of compounds the reductase superfamily can accept as substrates (Jörnvall et al., 1999). However, as in monoterpene metabolism, where rather similar reductive steps in the biosynthesis of menthol is performed by enzymes belonging to different reductase families, similar reductions with respect to substrate and mechanism in benzylisoquinoline metabolism are catalysed by enzymes belonging to at least two reductase families, the SDR and aldo-keto reductase families. As already discussed by Ringer et al. (2003) for monoterpene metabolism, it is obvious also for benzylisoquinoline biosynthesis that sequential reductase steps did not necessarily arise by gene duplication and diversification, but rather emerged from different ancestral sources.

### Nucleic Acid

The present application provides a nucleic acid encoding a polypeptide having salutaridine reductase activity.

The term "salutaridine reductase activity" refers the enzymatic activity of salutaridine reductase which catalyses the stereospecific reduction of the keto-group at position 7 of salutaridine to salutaridinol in morphin biosynthesis. The salutaridine reductase uses NADPH as cofactor and does not accept epi-salutaridinol as substrate. The salutaridine reductase is also able to catalyse the reverse reaction, from salutaridinol to salutaridine.

In certain embodiments, the nucleic acid of the present invention comprises or consists of the nucleotide sequence as set forth in SEQ ID NO: 1, comprises or consists a nucleotide sequence encoding the polypeptide as set forth in SEQ ID NO:2, or comprises or consists a nucleotide sequence encoding a polypeptide having the amino acid sequence as set forth in SEQ ID NO:2 further comprising an amino-terminal methionine.

In one embodiment, the nucleic acid of the present invention comprises or consists of a nucleotide sequence that is an ortholog of the nucleotide sequence as set forth in SEQ ID NO:2.

The term "ortholog" refers to a gene in a different species that has evolved from a common ancestor as the gene encoding salutaridine reductase of the present application.

In another embodiment, the nucleic acid of the present invention comprises or consists of a nucleotide sequence that is an allelic variant or a splice variant of the nucleotide sequence as set forth in SEQ ID NO:1.

The term "allelic variant" refers to one of several possible naturally occurring alternate forms of a gene occupying a given locus on a chromosome of an organism or a population of organisms.

The term "splice variant" refers to a nucleic acid molecule, usually RNA, which is generated by alternative processing of intron sequences in an RNA transcript.

In yet another embodiment, the nucleic acid of the present invention is a variant of the nucleic acid comprising or consisting of the nucleotide sequence of SEQ ID NO:1, or the nucleotide sequence encoding the polypeptide as set forth in SEQ ID NO:2, or the nucleotide sequence encoding a polypeptide having the amino acid sequence as set forth in SEQ ID NO:2 further comprising an amino-terminal methionine.

In one embodiment, the variant comprises or consists of a nucleotide sequence that is at least 60%, 70%, 80%, 85%, preferably at least 90%, 91%, 92%, 93%, 94%, more preferably at least 95%, 96%, 97%, 98%, even more preferably at least 99% identical to the nucleotide sequence of any of the nucleic acids as described above, in particular, the nucleotide sequence of SEQ ID NO:1, over its entire length.

In another embodiment, the variant comprises or consists of a nucleotide sequence encoding a polypeptide that has at least 60%, 70%, 80%, 85%, preferably at least 90%, 91 %, 92%, 93%, 94%, more preferably at least 95%, 96%, 97%, 98%, even more preferably at least 99% amino acid sequence identity or similarity to the polypeptide as set forth in SEQ ID NO:2 over its entire length.

The term "identity" as known in the art, refers to a relationship between the sequences of two or more polypeptide molecules or two or more nucleic acid molecules, as determined by comparing the sequences. In the art, "identity" also refers to the degree of sequence relatedness between polypeptide or nucleic acid molecule sequences, as the case may be, as determined by the match between strings of nucleotide or amino acid sequences. "identity" measures the percent of identical matches between two or more sequences with gap alignments addressed by a particular mathematical model of computer programs (i.e., "algorithms").

The term "similarity" is a related concept, but in contrast to "identity," refers to a measure of similarity which includes both identical matches and conservative substitution matches. Since conservative substitutions apply to polypeptides and not nucleic acid molecules, similarity only deals with polypeptide sequence comparisons. If two polypeptide sequences have, for example, 10 out of 20 identical amino acids, and the remainder are all non-conservative substitutions, then the percent identity and similarity would both be 50%. If in the same example, there are 5 more positions where there are conservative substitutions, then the percent identity remains 50%, but the percent similarity would be 75% (15 out of 20). Therefore, in cases where there are conservative substitutions, the degree of similarity between two polypeptide sequences will be higher than the percent identity between those two sequences.

Identity and similarity of related nucleic acid molecules and polypeptides can be readily calculated by known methods, including but not limited to those described in Computational Molecular Biology (A. M. Lesk, ed., Oxford University Press 1988), Biocomputing: Informatics and Genome Projects (D.W. Smith, ed., Academic Press 1993), Computer Analysis of Sequence Data (Part 1, A. M. Griffin and H. G. Griffin, eds., Humana Press 1994), Sequence Analysis in Molecular Biology (G. von Heijine, Academic Press 1987), Sequence Analysis Primer (M. Gribskov and J. Devereux, eds., M. Stockton Press 1991), and Carillo et al., SIAM J. Applied Math. 48: 1073 (1988).

Preferred methods to determine identity and/or similarity are designed to give the largest match between the sequences tested. Methods to determine identity and similarity are codified in publicly available computer programs.

Preferred computer program methods to determine identity and similarity between two sequences include, but are not limited to, the GCG program package, including GAP (Devereux et al., Nuc.Acids Res. 12: 387 (1984); Genetics Computer Group, University of Wisconsin, Madison, WI), BLASTP, BLASTN, and FASTA (Atschul et al., J. Mol.Biol. 215: 403-10 (1990)). The BLAST X program is publicly available from the National Center for Biotechnology Information (NCBI) and other sources (BLAST Manual (NCB NLM NIH,Bethesda, MD); Altschul et al., 1990, supra). The well-known Smith Waterman algorithm may also be used to determine identity.

By way of example, using the computer algorithm GAP (Genetics Computer Group), two polypeptides for which the percent sequence identity is to be determined are aligned for optimal matching of their respective amino acids (the "matched span" as determined by the algorithm). A gap opening penalty (which is calculated as 3X the average diagonal; the "average diagonal" is the average of the diagonal of the comparison matrix being used; the "diagonal" is the score or number assigned to each perfect amino acid match by the particular comparison matrix) and a gap extension penalty (which is usually 0.1X the gap opening penalty), as well as a comparison matrix such as PAM 250 or BLOSUM 62 are used in conjunction with the algorithm. A standard comparison matrix (see Dayhoff et al., 5 Atlas of Protein Sequence and Structure (Supp. 3 1978) for the PAM250 comparison matrix; see Henikoff et al., Proc. Natl. Acad. Sci. USA 89: 10915-19 (1992) for the BLOSUM 62 comparison matrix) is also used by the algorithm.

Preferred parameters for polypeptide sequence comparison include the following:
Algorithm: Needleman and Wunsch,J. Mol. Biol. 48: 443-53 (1970)
Comparison matrix: BLOSUM 62 from Henikoff et al., Proc. Natl. Acad. Sci. USA 89: 10915-19 (1992)
Gap Penalty: 12
Gap Length Penalty: 4
Threshold of Similarity: 0

The GAP program is useful with the above parameters. The aforementioned parameters are the default parameters for polypeptide comparisons (along with no penalty for end gaps) using the GAP algorithm.

Preferred parameters for nucleic acid molecule sequence comparison include the following:
Algorithm: Needleman et al., J. Mol Biol. 48: 443-53 (1970)
Comparison matrix: matches = +10, mismatch = 0
Gap Penalty: 50
Gap Length Penalty: 3

The GAP program is also useful with the above parameters. The aforementioned parameters are the default parameters for nucleic acid molecule comparisons.

Other exemplary algorithms, gap opening penalties, gap extension penalties, comparison matrices, thresholds of similarity, etc., may be used by those of skill in the art, including those set forth in the Program Manual, Wisconsin Package, Version 9, September, 1997. The particular choices to be made will depend on the specific comparison to be made, such as DNA to DNA, protein to protein, protein to DNA; and additionally, whether the comparison is between given pairs of sequences (in which case GAP or BestFit are generally preferred) or between one sequence and a large database of sequences (in which case FASTA or BLASTA are preferred).

In another embodiment, the variant comprises or consists of a nucleotide sequence which hybridizes under highly stringent or moderately stringent conditions to the nucleotide sequence as set forth in SEQ ID NO:1.

The conditions of high and moderate stringency are well known to those skilled in the art.

The term "highly stringent conditions" typically refers to those conditions that (1) employ low ion ic strength reagents and high temperature for washing, for example, 0.015M NaC1/0.0015 M sodium citrate/0.1%NaDodSO₄ (SDS) at 50°C, or (2) employ during hybridization a denaturing agent such as formamide, for example, 50% (vol/vol) formamide with 0.1% bovine serum albumin, 0.2% Ficoll, 0.1% polyvinylpyrrolidone, 50 mM sodium phosphate buffer (pH 6.5), 750 mM NaCl, and 75 mM sodium citrate at 42°C. Another example is the use of 50% formamide, 5X SSC (0.75 M NaCl, 0.075 M sodium citrate), 50 mM sodium phosphate (pH 6.8), 0.1 % sodium pyrophosphate, 5X Denhardt's solution, sonicated salmon sperm DNA (50µg/ml), 0.1% SDS, and 10% dextran sulfate at 42°C, with washes at 42°C in 0.2X SSC and 0.1 % SDS.

The term "moderately stringent conditions" typically refers to conditions which generally include the use of a washing solution and hybridization conditions (e.g., temperature, ionic strength, and percentage of SDS) less stringent than described above. An example of moderately stringent conditions are conditions such as overnight incubation at 37°C in a solution comprising 20% formamide, 5X SSC (150 mM NaCl, 15 mM trisodium citrate), 50 mM sodium phosphate (pH 7.6), 5X Denhardt's solution, 10% dextran sulfate, and 20 µl/ml denatured sheared salmon sperm DNA, followed by washing in 1X SSC at about 37-50°C. The skilled person will recognize how to adjust the temperature, ionic strength, etc., as necessary to accommodate factors such as probe length and the like.

In a further embodiment, the variant comprises or consists of a nucleotide sequence encoding a polypeptide as set forth in SEQ ID NO:2 with at least one modification selected from the group consisting of amino acid substitutions, amino acid insertions, amino acid deletions, carboxyl-terminal truncation, and amino-terminal truncation.

In particular, the variant comprises or consists of a nucleotide sequence encoding a polypeptide as set forth in SEQ ID NO:2 with least 1, 2, 3, 4, 5, 10, 15, 20 or 30 amino acid substitution(s), at least 1, 2, 3, 4, 5, 10, 15, 20 or 30 amino acid insertion(s), at least 1, 2, 3, 4, 5, 10, 15, 20 or 30 amino acid deletion(s), or with a carboxyl- and/or amino-terminal truncation of at least 1, 2, 3, 4, 5, 10, 15, 20, 30, 50, 100, 150, 200, 250 or 300 amino acid(s).

The term "amino acid substitution" refers to both conservative amino acid substitution and non-conservative amino acid substitution.

The term "conservative amino acid substitution" refers to a substitution of a native amino acid residue with a non-native residue such that there is little or no effect on the size, polarity or charge of the amino acid residue at that position. For example, a conservative substitution results from the replacement of a non-polar residue in a polypeptide with any other non-polar residue. Furthermore, any native residue in the polypeptide may also be substituted with alanine, as has been previously described for "alanine scanning mutagenesis" (Cunnigham et al., Science 244: 1081-85 (1989)). General rules for conservative amino acid substitutions are set forth in Table 1.

**Table 1. Conservative Amino Acid Substitutions**

| Original Residue | Exemplary Substitutions | Preferred Substitution |
|---|---|---|
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, Gln, Asn | Arg |
| Met | Leu, Phe, Ile | Leu |
| Phe | Leu, Val, Ile, Ala, Tyr | Leu |
| Pro | Ala | Ala |
| Ser | Thr | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

Conservative amino acid substitutions also encompass non-naturally occurring amino acid residues that are typically incorporated by chemical peptide synthesis rather than by synthesis in biological systems. These include peptidomimetics, and other reversed or inverted forms of amino acid moieties.

The term "non-conservative amino acid substitution" refers to a substitution of a native amino acid residue with a non-native residue such that there is change in the size, polarity or charge of the amino acid residue at that position. For example, a non-conservative substitution results from the replacement of a non-polar residue in a polypeptide with a polar residue.

Non-conservative amino acid substitutions may alter (a) the structure of the molecular backbone in the area of the substitution, for example, as a sheet or helical conformation, (b) the charge or hydrophobicity of the molecule at the target site, or (c) the bulk of the side chain.

Naturally occurring residues may be divided into groups based on common side chain properties:
1) hydrophobic: norleucine, Met, Ala, Val, Leu, lie;
2) neutral hydrophilic: Cys, Ser, Thr;
3) acidic: Asp, Glu;
4) basic: Asn, Gln, His, Lys, Arg;
5) residues that influence chain orientation: Gly, Pro; and
6) aromatic: Trp, Tyr, Phe.

Non-conservative amino acid substitutions may involve the exchange of a member of one of these classes for a member from another class.

The nucleic acid of the present invention also includes a nucleic acid which is a fragment of any of the nucleic acids described above.

In one embodiment, the fragment comprises or consists of at least 15, 30, 45, 60, 75, 90, 120, 150, 180, 210, 240, 270, 300, 450, 600, 750 or 900 consecutive nucleotides of the nucleic acid of the present invention, preferably, the nucleic acid having the nucleotide sequence as set forth in SEQ ID NO:1.

In another embodiment, the fragment comprises or consists of a nucleotide sequence encoding a polypeptide of at least 5, 10, 15, 20, 25, 30, 40, 50, 60, 70, 80, 90, 100, 150, 250 or 300 amino acid residues.

In yet another embodiment, the fragment comprises or consists of a nucleotide sequence encoding at least 5, 10, 15, 20, 25, 30, 40, 50, 60, 70, 80, 90, 100, 150, 250 or 300 consecutive amino acid residues of any of the polypeptides encoded by the nucleic acid of the present invention, preferably, the polypeptide as set forth in SEQ ID NO:2.

The nucleic acid of the present application also includes a nucleic acid comprising or consisting of a nucleotide sequence that is complementary to any of the nucleic acids described above.

In the context of the present invention, "complementary" means that Watson-Crick base-pairs can form between a majority of the bases in the complementary sequence and the reference sequence. Preferably, the complementarity is 100%, but at least 1, 2, 3, 4 or 5 mismatches can be tolerated.

In one embodiment, the complementary nucleic acid is at least 15, 19, 21, 23, 25, 30, 40 or 50 nucleotides in length.

In another embodiment, the complementary nucleic acid further comprises one or more stretches of non-complementary sequence(s).

In a preferred embodiment, the complementary nucleic acid is an antisense oligonucleotide. In another preferred embodiment, the complementary nucleic acid is an siRNA. In yet another preferred embodiment, the complementary nucleic acid is a ribozyme. In a further preferred embodiment, the complementary nucleic acid is an shRNA.

The nucleic acid of the present invention may be single-stranded or double-stranded. The nucleic acid of the present invention may be DNA or RNA. The nucleic acid of the present invention may contain naturally occurring or modified nucleotides, or any of the known base analogs of DNA and RNA such as, but not limited to 4acetylcytosine, 8-hydroxy-N6-methyladenosine, aziridinyl-cytosine, pseudoisocytosine, 5- (carboxyhydroxylmethyl) uracil, 5-fluorouracil, 5bromouracil, 5-carboxymethylaminomethyl-2-thiouracil, 5-carboxymethylaminomethyluracil, dihydrouracil, inosine, N6-iso-pentenyladenine, methyladenine, 1-methylpseudouracil, 1-methylguanine, 1-methylinosine, 2,2dimethylguanine,2-methyladenine, 2-methylguanine,3-methylcytosine, 5methylcytosine, N6-methyladenine, 7-methylguanine, 5methylaminomethyluracil, 5-methoxyamino-methyl-2-thiouracil, beta-Dmannosylqueosine, 5'-methoxycarbonyl-methyluracil, 5-methoxyuracil, 2methylthio-N6-isopentenyladenine, uracil-5-oxyacetic acid methylester, uracil-5oxyacetic acid, oxybutoxosine, pseudouracil, queosine, 2-thiocytosine, 5-methyl2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine. Furthermore, the nucleic acid of the present invention may be modified covalently or non-covalently, resulting in derivatives of the nucleic acid of the present invention.

In a preferred embodiment, the nucleic acid of the present invention is an isolated nucleic acid.

The term "isolated nucleic acid" refers to a nucleic acid that is removed from its natural environment such as the genomic DNA and that is free from at least one contaminating nucleic acid with which it is naturally associated, and preferably substantially free from any other contaminating nucleic acid which would interfere with its use in protein production or other use.

Recombinant DNA methods used herein are generally those set forth in Sambrook et al., Molecular Cloning : A Laboratory Manual (Cold Spring Harbor Laboratory Press, 1989) and/or Current Protocols in Molecular Biology (Ausubel et al., eds., Green Publishers Inc. and Wiley and Sons 1994).

The nucleic acid of the present invention may be obtained by hybridization screening of a genomic or cDNA library, or by PCR amplification. Typically, conditions of moderate or high stringency will be employed for screening to minimize the number of false positives obtained from the screen.

The nucleic acid of the present invention may also be identified by expression cloning which employs detection of positive clones based upon a property of the expressed protein.

The nucleic acid of the present invention may also be chemically synthesized using methods well known to a skilled person such as those described by Engels et al., Angew. Chem. Intl. Ed. 28: 716-34 (1989). These methods include, inter alia, the phosphotriester, phosphoramidite, and H-phosphonate methods for nucleic acid synthesis. A preferred method for such chemical synthesis is polymer-supported synthesis using standard phosphoramidite chemistry.

Variants of the nucleic acid molecule of the present invention may be produced using site directed mutagenesis, PCR amplification, or other appropriate methods (see Sambrook et al., supra, and Ausubel et al., supra, for descriptions of mutagenesis techniques). Chemical synthesis using methods described by Engels et al., supra, may also be used to prepare such variants. Other methods known to the skilled person may be used as well.

The nucleic acid of the present invention may be used to modulate (increase or decrease) the expression level of salutaridine reductase in a host cell or a host organism, whereby modulating the production of morphinan alkaloids, in particular, morphine.

A "morphinan" is a chemical that contains the following ring system in its structure.

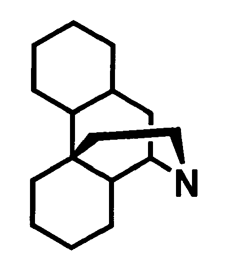

In opium poppy specifically, all biosynthetic intermediates from salutaridine to morphine are considered to be morphinans. The occurrence of morphinans is, however, not restricted to opium poppy.

### Vector

The present invention provides an expression vector comprising the nucleic acid (including the complementary nucleic acid) of the present invention, operably linked to transcription regulatory sequences.

In one embodiment, the transcription regulatory sequences are heterologous transcription regulatory sequences which are different from the native transcription regulatory sequences for salutaridine reductase.

The term "transcription regulatory sequences" refers to elements that are invovled in the regulation (positive or negative) of transcription, including but not limited to promoter and enhancer.

The term "heterologous transcription regulatory sequences" refer to transcription regulatory sequences which are not the same as the native transcription regulatory sequences for salutaridine reductase of the present invention. The heterologous transcription regulatory sequences may be from one or more different genes in the same organsim as the salutaridine reductase of the present application; the heterologous transcription regulatory sequences may also be from one or more different organsim(s) as the salutaridine reductase of the present application.

The term "expression vector" refers to a vector that is suitable for propagation in a host cell and contains nucleic acid sequences that direct and/or control the expression of inserted heterologous nucleic acid sequences. Expression includes, but is not limited to, processes such as transcription, translation, and RNA splicing if introns are present.

The vector is typically selected to be functional in the particular host cell employed, i.e., the vector is compatible with the host cell machinery such that amplification of the gene and/or expression of the gene can occur.

Typically, expression vectors used in any of the host cells will contain sequences for plasmid maintenance and for cloning and expression of exogenous nucleotide sequences. Such sequences, collectively referred to as "flanking sequences" typically include one or more of the following: a promoter, one or more enhancer sequences, an origin of replication, a transcriptional termination sequence, a complete intron sequence containing a splice donor and acceptor, a leader sequence for secretion, a ribosome binding site, a polyadenylation sequence, a polylinker region for inserting the nucleic acid encoding the polypeptide to be expressed, and a selectable marker element. Optionally, the vector may contain a "tag" sequence.

A tag sequence is typically an oligonucleotide sequence located at the 5' or 3' end of the nucleic acid encoding the polypeptide to be expressed. The oligonucleotide sequence may encode poly-His (such as hexa-His), or other tags such as FLAG, HA (hemaglutinin of Influenza virus) or myc for which commercially available antibodies exist. This tag is typically fused to the polypeptide of interest upon expression, and can serve as a means for affinity purification of the polypeptide from the host cell and detection. Affinity purification can be accomplished, for example, by column chromatography using antibodies against the tag as an affinity matrix. Optionally, the tag can subsequently be removed from the purified polypeptide by various means such as using certain peptidases for cleavage.

Flanking sequences may be homologous (i.e., from the same species and/or strain as the host cell), heterologous (i.e., from a species other than the host cell species or strain), hybrid (i.e., a combination of flanking sequences from more than one source), synthetic, or native sequences which normally function to regulate salutaridine reductase expression. As such, the source of flanking sequences may be any prokaryotic or eukaryotic organism, any vertebrate or invertebrate organism, or any plant, provided that the flanking sequences is functional in, and can be activated by, the host cell machinery.

The flanking sequences useful in the vectors of the present invention may be obtained by any of the methods well known in the art.

An origin of replication is typically a part of prokaryotic expression vectors purchased commercially, and aids in the amplification of the vector in a host cell. Amplification of the vector to a certain copy number can, in some cases, be important for optimal expression of the polypeptide to be expressed. If the vector of choice does not contain an origin of replication, one may be chemically synthesized based on a known sequence, and ligated into the vector.

The origin of replication from the plasmid pBR322 (Product No. 303-3s, New England Biolabs, Beverly, MA) is suitable for most Gram-negative bacteria; various viral origins, such as that of SV40, polyoma, adenovirus, vesicular stomatitus virus (VSV) or papilloma viruses such as HPV or BPV, are useful for cloning vectors in mammalian cells. Generally, the origin of replication is not needed for mammalian expression vectors. While the origin of replication is easily cloned from a library or even purchased commercially as part of a vector, it can also be readily synthesized using methods for nucleic acid synthesis such as those described above.

A selectable marker gene encodes a protein necessary for the survival and growth of a host cell grown in a selective culture medium. Typical selection marker genes encode proteins that (a) confer resistance to antibiotics or other toxins, for example, ampicillin, tetracycline, or kanamycin for prokaryotic host cells, (b) complement auxotrophic deficiencies of the cell; or (c) supply critical nutrients not available from the media. Preferred selectable markers include, but are not limited to, the kanamycin resistance gene, the ampicillin resistance gene, the tetracycline resistance gene, and the hygromycin resistance gene. A neomycin resistance gene may be used for selection in both prokaryotic and eukaryotic host cells.

Other selection genes may be used to amplify the gene that will be expressed. Amplification is the process wherein genes that are in greater demand for the production of a protein critical for growth are reiterated in tandem within the chromosomes of successive generations of recombinant cells. Examples of suitable selectable markers for mammalian cells include, but are not limited to, dihydrofolate reductase (DHFR) and thymidine kinase. The mammalian cell transformants are placed under selection pressure that only the transformants are uniquely adapted to survive by virtue of the marker present in the vector. Selection pressure is imposed by culturing the transformed cells under conditions in which the concentration of selection agent in the medium is successively changed, thereby leading to amplification of both the selection gene and the DNA that encodes the polypeptide to be expressed. As a result, increased quantities of the polypeptide of interest are synthesized from the amplified DNA.

A ribosome binding site is usually necessary for translation initiation of mRNA and is characterized by a Shine-Dalgarno sequence in prokaryotic cells or a Kozak sequence in eukaryotic cells. The ribosome binding site is typically located 3' to the promoter and 5' to the coding sequence of the polypeptide to be expressed. The Shine-Dalgarno sequence is varied but is typically a polypurine (i.e., having a high A-G content). Many Shine-Dalgarno sequences have been identified, each of which can be readily synthesized using methods set forth above and used in a prokaryotic vector.

A leader, or signal, sequence may be used to direct the polypeptide to be expressed out of the host cell. Typically, the signal sequence is positioned in the coding region of the nucleic acid encoding the polypeptide to be expressed, or directly at the 5' end of the coding region. Many signal sequences have been identified, and any of them that are functional in the selected host cell may be used in conjunction with the nucleic acid of the present invention. A signal sequence may be homologous (naturally occurring) or heterologous to the nucleic acid of the present invention, Additionally, a signal sequence may be chemically synthesized using methods set forth above. In most cases, secretion of the polypeptide of interest from the host cell via the presence of a signal peptide will result in the removal of the signal peptide from the polypeptide.

In some cases, such as where glycosylation is desired in a eukaryotic host cell expression system, one may manipulate the various presequences to improve glycosylation and/or yield. For example, one may alter the peptidase cleavage site of a particular signal peptide, or add prosequences, which also may affect glycosylation. The final protein product may have, in the -1 position (relative to the first amino acid of the mature protein) one or more additional amino acids incident to expression, which may not have been totally removed. For example, the final protein product may have one or two amino acid found in the peptidase cleavage site, attached to the N-terminus. Alternatively, use of some enzyme cleavage sites may result in a slightly truncated form of the desired polypeptide, if the enzyme cuts at such area within the mature polypeptide.

In many cases, transcription of a nucleic acid molecule is increased by the presence of one or more introns in the vector; this is particularly true where a polypeptide is produced in eukaryotic host cells, especially mammalian host cells.

The introns used may be naturally occurring especially where the gene used is a full-length genomic sequence or a fragment thereof.

Where the intron is not naturally occurring within the gene (as for most cDNAs), the intron may be obtained from another source. The position of the intron with respect to flanking sequences and the gene to be expressed is generally important, as the intron must be transcribed to be effective. Thus, when a cDNA molecule is being expressed, the preferred position for the intron is 3' to the transcription start site and 5' to the poly-A transcription termination sequence.

Preferably, the intron or introns will be located on one side (i.e., 5' or 3') of the cDNA such that it does not interrupt the coding sequence. Any intron from any source, including any viral, prokaryotic and eukaryotic (plant or animal) organisms, may be used to practice this invention, provided that it is compatible with the host cell into which it is inserted. Also included herein are synthetic introns. Optionally, more than one intron may be used in a vector.

The expression and cloning vectors of the present invention will typically contain a promoter that is recognized by the host organism and operably linked to the nucleic acid of the present invention. Promoters are untranslated sequences located upstream (i.e., 5') to the start codon of a structural gene (generally within about 100 to 1000bp) that control the transcription and translation of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or chemical or a change in temperature. A typical inducible system which functions in bacteria is the lac system where transcription is inactivated by the lac repressor until the inhibition is relieved by the addition of IPTG; and a typical inducible system which functions in both bacteria and eukaryotic cells is the tet-on and the tet-off system.

A large number of promoters, recognized by a variety of potential host cells, are well known. A heterologous promoter which is not the native prompter of salutaridine reductase is preferred if it permits greater transcription and higher yields of the expressed protein as compared to the native promoter, and if it is compatible with the host cell system that has been selected for use.

Promoters suitable for use with prokaryotic hosts include, but are not limited to, the beta-lactamase and lactose promoter systems; alkaline phosphatase, a tryptophan (trp) promoter system; and hybrid promoters such as the tac promoter. Other known bacterial promoters are also suitable. Their sequences have been published, thereby enabling one skilled in the art to ligate them to the desired DNA sequence, using linkers or adapters as needed to supply any required restriction sites.

Suitable promoters for use with yeast hosts are also well known in the art. Yeast enhancers are advantageously used with yeast promoters.

Suitable promoters for use with mammalian host cells are well known in the art and include, but are not limited to, those obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40). Other suitable mammalian promoters include heterologous mammalian promoters, for example, heat-shock promoters and the actin promoter.

Additional promoters which may be of interest include, but are not limited to, the SV40 early promoter region (Bemoist and Chambon, Nature 290: 304-10 (1981)); the CMV promoter; the promoter contained in the 3' long terminal repeat of Rous sarcoma virus (Yamamoto, et al.,Cell 22: 787-97 (1980)); the herpes thymidine kinase promoter (Wagner et al., Proc.Natl. Acad. Sci. U. S. A. 78:1444-45 (1981)); the regulatory sequences of the metallothionine gene (Brinster et al., Nature 296: 39-42 (1982)); prokaryotic expression vectors such as the beta-lactamase promoter (Villa Kamaroff et al., Proc. Natl. Acad. Sci. U. S. A., 75: 3727-31 (1978)); or the tac promoter (DeBoer et al., Proc.Natl. Acad. Sci. U. S. A., 80: 21-25 (1983)).

In a preferred embodiment of the present invention, the promoter is one that is functional in plant cells, including, but not limited to, cauliflower mosaic virus 35S promoter, subterranean clover virus S4 and S7 promoters. The promoters may be placed in tandem, for example, to produce a 35S35S promoter or an S4S4 or S7S7 promoter.

An enhancer sequence may be inserted into the vector to increase the transcription of a nucleic acid of the present invention by higher eukaryotes. Enhancers are cis-acting elements of DNA, usually about 10-300bp in length, that act on the promoter to increase its transcription. Enhancers are relatively orientation and position independent. They have been found 5' and 3' to the transcription unit. Several enhancer sequences available from mammalian genes are known (e.g., globin, elastase, albumin, alpha-feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters. While an enhancer may be spliced into the vector at a position 5' or 3' to the coding seqeuence, it is typically located at a site 5' from the promoter.

The term"operably linked" refers to an arrangement of flanking sequences wherein the flanking sequences so described are configured or assembled so as to perform their usual function. Thus, a flanking sequence operably linked to a coding sequence may be capable of effecting the replication, transcription and/or translation of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence and the promoter sequence can still be considered "operably linked" to the coding sequence.

Expression vectors of the invention may be constructed from starting vectors such as a commercially available vector. Such vectors may or may not contain all of the desired flanking sequences. Where one or more of the flanking sequences set forth above are not already present in the vector to be used, they may be individually obtained and ligated into the vector. Methods used for obtaining each of the flanking sequences are well known to one skilled in the art.

Preferred vectors for practicing this invention are those which are compatible with bacterial, yeast, or plant host cells. Such vectors include, but are not limited to pART27, pPLEXX002.

The vector of the present invention may be used to modulate (increase or decrease) the expression level of salutaridine reductase in a host cell or a host organism, whereby modulating the production of morphinan alkaloids, in particular, morphine.

### Host Cell

The present invention provides a provides a host cell comprising the expression vector of the present invention.

The host cell of the present invention may be selected from a prokaryotic cell and a eukaryotic cell. Prokaryotic host cells include, but are not limited to, bacterial cells such as various strains of *E. coli, B. subtilis, Pseudomonas spp.,* other *Bacillus spp., Streptomyces spp.* Eukaryotic host cells include, but are not limited to yeast, plant cells, invertebrate cells and vertebrate cells. Invertebrate cells include insect cells; vertebrate cells include mammalian cells. Preferred yeast cells include, but are not limited to, *Saccharomyces cerivisae* and *Schizosaccharomyces pombe.* Preferred mammalian cells include, but are not limited to, CHO, COS, 3T3, HeLa, and 293.

In a preferred embodiment, the host cell is a bacterial cell, in particular an *E*. *coli.*

In another preferred embodiment, the host cell is a plant cell, preferably a cell of a plant belonging to the genus *Papaver,* more preferably a cell of a plant belonging to a species selected from *Papaver somniferum, Papaver bracteatum, Papaver cylindricum, Papaver orientale, Papaver setigerum, Papaver pseudo-orientale, Papaver lauricola, Papaver persicum, Papaver caucasium, Papaver carmeli, Papaver arenarium, Papaver argemone, Papaver atlanticum, Papaver commutatum, Papaver dubium, Papaver fugax, Papaver hybridum, Papaver lateritium, Papaver oreophilum, Papaver pavonium, Papaver pilosum, Papaver pyrenaicum,* even more preferably a cell of a plant which naturally produce morphinan alkaloids, such as *Papaver somniferum, Papaver bracteatum* and *Papaver setigerum.*

A host cell of the present invention may be obtained by introducing an expression vector of the present invention into a host cell via any of the methods well known in the art, including, but not limited to, transformation (e.g., heat shock, electroporation), transfection (e.g., calcium phosphate precipitation, electroporation, lipofection, the DEAE-dextran method), infection or transduction (e.g., by bacteriophage or viruses), and microinjection. The method will at least in part depend on the type of host cell to be used. These and other suitable methods are well known to a skilled person, and are set forth, for example, in Sambrook et al., supra.

In certain embodiments, the nucleic acid of the present invention contains codons which have been altered for optimal expression in a given host cell. Particular codon alterations will depend upon the host cell selected for expression. Codon optimization can be carried out by a variety of methods, for example, by selecting codons which are preferred for use in highly expressed genes in a given host cell. Computer algorithms which incorporate codon frequency tables are provided by the University of Wisconsin Package Version 9.0, Genetics Computer Group, Madison, Wl.

The host cell of the present invention may be used to produce salutaridine reductase which may be used for the *in vitro* production of morphinan alkaloids, in particular, salutaridinol and morphine. The host cell of the present invention may also be directly used for the production of morphinan alkaloids.

### Transgenic Plant

The present invention provides a transgenic plant comprising the nucleic acid (including the complementary nucleic acid) or the vector of the present invention.

In a perferred embodiment, the nucleic acid or the vector is stably integrated into the genome of the plant.

In one embodiment, the transgenic plant belongs to the genus *Papaver,* perfereably to a species selected from *Papaver somniferum, Papaver bracteatum, Papaver cylindricum, Papaver orientale, Papaver setigerum, Papaver pseudo-orientale, Papaver lauricola, Papaver persicum, Papaver caucasium, Papaver carmeli, Papaver arenarium, Papaver argemone, Papaver atlanticum, Papaver commutatum, Papaver dubium, Papaver fugax, Papaver hybridum, Papaver lateritium, Papaver oreophilum, Papaver pavonium, Papaver pilosum, Papaver pyrenaicum,* even more preferably to a species which naturally produce endogenous morphinan alkaloids, such as *Papaver somniferum, Papaver bracteatum* and *Papaver setigerum.*

In certain embodiments, the transgenic plant of the present invention has increased expression of salutaridine reductase and consequently increased production of morphinan alkaloids as compared to non-trangenic plant of the same origin. In other embodiments, the transgenic plant of the present invention has decreased expression of salutaridine reductase and consequently decreased production of morphinan alkaloids as compared to non-trangenic plant of the same origin.

The present invention also provides parts of the transgenic plant of the present invention, including, but not limited to, seed, seed pod or straw.

The transgenic plant and the transgenic plant part of the present invention may be used to produce morphinan alkaloids, in particular, morphine. The transgenic plant and the transgenic plant part of the present invention may also be used for the preparation of a medicament for reducing and/or eliminating pain and/or reducing and/or suppressing coughing.

### Polypeptide

The present invention provides a polypeptide having salutaridine reductase activity. The polypeptide of the present invention is encoded by a nucleic acid of the present invention which encodes a polypeptide having salutardin reductase activity.

In one embodiment, the polypeptide of the present invention comprises or consists of the amino acid sequence as set forth in SEQ ID NO:2, optionally further comprising an amino-terminal methionine.

In another embodiment, the polypeptide of the present invention is an orthologous polypeptide of the amino acid sequence as set forth in SEQ ID NO:2.

In yet another embodiment, the polypeptide of the present invention is encoded by a nucleic acid which is an allelic variant or a splice variant of the nucleotide sequence as set forth in SEQ ID NO:1.

In a further embodiment, the polypeptide of the present invention is a variant of the polypeptide comprising or consisting of the amino acid sequence as set forth in SEQ ID NO:2, or the polypeptide encoded by the nucleic acid comprising or consisting of the nucleotide sequence of SEQ ID NO:1.

In one embodiment, the variant is encoded by a nucleotide sequence that is at least 60%, 70%, 80%, 85%, preferably at least 90%, 91%, 92%, 93%, 94%, more preferably at least 95%, 96%, 97%, 98%, even more preferably at least 99% identical to the nucleotide sequence of SEQ ID NO:1 over its entire length.

In another embodiment, the variant has at least 60%, 70%, 80%, 85%, preferably at least 90%, 91%, 92%, 93%, 94%, more preferably at least 95%, 96%, 97%, 98%, even more preferably at least 99% amino acid sequence identity or similarity to the polypeptide as set forth in SEQ ID NO:2 over its entire length.

In a further embodiment, the variant is encoded by a nucleic acid comprising or consisting of a nucleotide sequence which hybridizes under highly stringent or moderately stringent conditions to the nucleotide sequence as set forth in SEQ ID NO:1.

In a still further embodiment, the variant comprises or consists of an amino acid sequence as set forth in SEQ ID NO:2 with at least one modification selected from the group consisting of amino acid substitutions, amino acid insertions, amino acid deletions, carboxyl-terminal truncation, and amino-terminal truncation.

In particular, the variant comprises or consists of an amino acid sequence as set forth in SEQ ID NO:2 with least 1, 2, 3, 4, 5, 10, 15, 20 or 30 amino acid substitution(s), at least 1, 2, 3, 4, 5, 10, 15, 20 or 30 amino acid insertion(s), at least 1, 2, 3, 4, 5, 10, 15, 20 or 30 amino acid deletion(s), or with a carboxyl- and/or amino-terminal truncation of at least 1, 2, 3, 4, 5, 10, 15, 20, 30, 50, 100, 150, 200, 250 or 300 amino acid(s).

In certain embodiments, the polypeptide of the present invention is a fragment of any of the polypeptides described above.

In one embodiment, the fragment is encoded by a nucleic acid comprising or consisting of at least 15, 30, 45, 60, 75, 90, 120, 150, 180, 210, 240, 270, 300, 450, 600, 750 or 900 consecutive nucleotides of the nucleic acid of the present invention, preferably, the nucleic acid having the nucleotide sequence as set forth in SEQ ID NO: 1.

In another embodiment, the fragment is a polypeptide of at least 5, 10, 15, 20, 25, 30, 40, 50, 60, 70, 80, 90, 100, 150, 250 or 300 amino acid residues.

In yet another embodiment, the fragment comprises or consists of at least 5, 10, 15, 20, 25, 30, 40, 50, 60, 70, 80, 90, 100, 150, 250 or 300 consecutive amino acid residues of any of the polypeptides encoded by the nucleic acids of the present invention, preferably, the polypeptide as set forth in SEQ ID NO:2.

The polypeptide of the present invention can also be a fusion polypeptide comprising or consisting of any of the polypeptides described above fused to a heterologous amino acid sequence.

The term "a heterologous amino acid sequence" refers to an amino acid sequence that is not part of the amino acid sequence of the naturally occuring salutaridine reductase.

The heterologous amino acid sequence may be an epitope tag which may be useful in the purification and the detection of the fusion polypeptide. Commonly used epitope tags include, but are not limited to, His tag (poly-His or hexa-His), Myc tag, FLAG tag, and HA tag.

The heterologous amino acid sequence may also be Fc domain of an immunoglobulin (i.e., antibody) molecule or any other sequence which may be useful in the purification and the detection of the fusion polypeptide.

The heterologous amino acid sequence may also be sequences that are involved in the dimerization or multimerization of the fusion protein, including, but not limited to, the leucine zipper sequences and the Fc domain of an antibody.

The heterologous amino acid sequences may also be sequences that are capable of targeting the fusion protein to particular cells or tissues or facilitating the transport of the fusion protein across the cell membrane.

The polypeptide of the present invention includes a derivative of any of the polypeptide described above. The term "derivative" refers to a chemically modified polypeptide, for example, by covalent attachment of one or more polymers, including, but not limited to, water soluble polymers, N-linked or O-linked carbohydrates, sugars, phosphates, and/or other molecules. The derivatives are modified in a manner that is different from naturally occurring polypeptide, either in the type or location of the molecules attached to the polypeptide. Derivatives further include the deletion of one or more chemical groups naturally attached to the polypeptide.

In a preferred embodiment, the polypeptide of the present invention is an isolated polypeptide. The term "isolated polypeptide" refers to a polypeptide this is removed from its natural environment such as a cell and that is free from at least one contaminating polypeptide that is found in its natural environment, and preferably substantially free from any other contaminatin polypeptides which would interfere with its use.

The polypeptide of the present invention may be obtained from naturally occurring plants which express the polypeptide endogenously, or host cells or transgenic plant or plant part of the present invention cultured/grown under conditions which allow the expression of the polypeptide.

Polypeptides, in particular, fragments, of the present invention may also be prepared by chemical synthesis methods (such as solid phase peptide synthesis) using techniques known in the art such as those set forth by Merrifield et al. (J. Am. Chem. Soc. 85: 2149 (1963)), Houghten et al. (Proc Natl. Acad. Sci. USA 82: 5132 (1985)), and Stewart and Young, Solid Phase Peptide Synthesis (Pierce Chemical Co. 1984). The polypeptide may be synthesized with or without a methionine on the amino terminus.

The polypeptide of the present invention may be used in the *in vitro* production of morphinan alkaloids, in particular, salutaridinol and/or morphine.

### Antibody

The present invention provides an antibody or a fragment thereof that specifically binds to the polypeptide of the present invention.

The antibody of the present invention may be a polyclonal antibody, a monoclonal antibody, a chimeric antibody, or a single chain antibody.

The antibody fragment of the present invention may be an Fab fragment, an F(ab')2 fragment, or any other fragment which retains the antigen binding specificity of the intact antibody.

The present invention also provides a hybridoma that produces the monoclonal antibody of the present invention.

The antibody and fragment thereof and the hybridoma of the present invention can be obtained by any of the methods known in the art, for example, those described in the Current Protocols in Immunology (Coligan JE et al. ed., Wiley and Sons).

The antibody and fragment thereof of the present invention may be used for the purification, the detection and quantitation, and/or the sequestration of salutaridine reductase.

### Method for Polypeptide Detection

The present invention provides a method for detecting and/or quantitating the amount of the polypeptide of the present invention, comprising the steps of:
(a) contacting a sample with the antibody or a fragment thereof of the present invention;
(b) detecting the complex formed between the polypeptide and the antibody or a fragment thereof; and
(c) optionally quantitating the amount of the complex.

In certain embodiments, the sample is a cell lysate of a naturally occurring plant or a part thereof, or a host cell or a transgenic plant or a part thereof of the present invention. The cell lysate may be a whole cell lysate, or may be a cytoplasmic fraction.

Any assay format known in the art may be used, such as western blotting, dot blotting, ELISA and plate assay. These and other methods are described in Current Protocols in Protein Science (Coligan JE et al. ed., Wiley and Sons).

### Method for Polypeptide Production

The present invention also provides a process for producing the polypeptide of the present invention comprising culturing the host cell of the present invention under suitable conditions to express the polypeptide, and optionally isolating the polypeptide from the culture.

Host cells comprising an expression vector comprising a nucleic acid encoding a polypeptide of the present invention may be cultured using standard media well known to a skilled person. The media will usually contain all nutrients necessary for the growth and survival of the cells. Suitable media for culturing *E. coli* cells include, but are not limited to, Luria Broth (LB) and Terrific Broth (TB). Suitable media for culturing plant cells include, but are not limited to, LS (Linsmaier and Skoog), MS (Murashige and Skoog), B5, B5O, and Anderson. Suitable media for culturing mammalian cells include, but are not limited to, RPMI 1640, MEM, DMEM, IMDM, all of which may be supplemented with serum and/or growth factors as required by the particular cell line being cultured. Suitable media for culturing insect cells include, but are not limited to, Grace's medium supplemented with yeastolate, lactalbumin hydrolysate, and/or fetal calf serum as necessary.

Typically, an antibiotic or other compound useful for the selective growth of transfected or transformed cells is added as a supplement to the media. The compound to be used will be dictated by the selectable marker present on the expression vector contained in the host cell. For example, where the selectable marker is kanamycin resistance, the compound added to the culture medium will be kanamycin. Other compounds for selective growth include, but are not limited to, ampicillin, tetracycline, neomycin, and hygromycin.

Furthermore, when an inducible expression system is employed, a compound that induces expression should be included in the culture media, and/or a compound that represses expression should be excluded or removed from the culture media. For example, IPTG is supplied to *E. coli* carrying the lac inducible system to activate transcription of the heterologous gene to be expressed. In another example, a tetracycline derivative, doxycycline is supplied to cells carrying the tet-on system to activate trasncription; whereas tetracycline or doxycline is removed from cells carrying the tet-off system to activate transcription.

The polypeptide of the present invention may be enriched, partially purified or purified from the cultured host cells or the culture media by any suitable methods known in the art, such as those described in the Current Protocol in Protein Science (supra).

When the polypeptide of the present invention is secreted from the host cell, it can be purified directly from the culture supernatant. When the polypeptide of the present invention is produced intracellularly in the host cell, the intracellular material (including inclusion bodies of gram-negative bacteria) can be extracted from the host cell using any standard technique known to a skilled person. For example, the host cells can be lysed to release the contents of the periplasm/cytoplasm by French press, homogenization, and/or sonication followed by centrifugation.

When an antibody is available, the polypeptide of the present invention may be purified using an affinity column containing an antibody or a fragment thereof of the present invention.

When the polypeptide of the present invention is expressed as a fusion polypeptide comprising a tag, the polypeptide may be purified using an affinity column containing a molecule that bind specifically to the tag. In the case of the myc tag, FLAG tag, and/or the HA tag, the specific binding molecule may be an antibody or a fragment thereof. In the case of the His tag, the specific binding molecule may be nickel or cobalt.

When the polypeptide of the present invention is expressed as a fusion polypeptide comprising a Fc or any other sequence to which a specific binding partner exists, the polypeptide may be purified using an affinity column containing the specific binding partner. In certain embodiment, the specific binding partner is an antibody or a fragment thereof.

When the polypeptide of the present invention is expressed without a tag attached, and no antibody is available, other well-known procedures for polypeptide purification can be used. Such procedures include, without limitation, ion exchange chromatography, molecular sieve chromatography, HPLC, native gel electrophoresis in combination with gel elution, and preparative isoelectric focusing ("Isoprime" machine/technique, Hoefer Scientific). In some cases, two or more of these techniques may be combined to achieve increased purity.

In some cases, the polypeptide of the present invention may not be biologically active upon isolation. Various methods known in the art for "refolding" or converting the polypeptide to its tertiary structure and generating disulfide linkages can be used to restore biological activity.

The same methods may be used to isolate the polypeptide of the present invention from the transgenic plant or a part thereof of the present invention.

### Method for Producing Transgenic Plant, Plant Part or Plant Cell

The present invention provides a method for producing a plant or a plant part having altered (increased or decreased) alkaloid production, comprising the steps of:
(a) introducing the nucleic acid of the present invention into a cell or a part of an alkaloid-producing plant;
(b) growing the product of (a) under suitable conditions which permit the expression of the nucleic acid; and
(c) optionally regenerating a whole plant from said cell or part.

The present invention also provides a method for producing a plant cell culture having altered (increased or decreased) alkaloid production, comprising the steps of:
(a) introducing the nucleic acid of the present invention into a cell of an alkaloid-producing plant;
(b) growing the product of (a) under suitable conditions which permit the expression of the nucleic acid; and
(c) establishing a cell culture from said cell.

The term "alkaloid-producing plant" refers to any plants that naturally produce alkaloids utilizing endogenous enzymes. The alkaloids may be morphinan or non-morphinan alkaloids. In a preferred embodiment, the alkaloids are morphinan alkaloids.

In one embodiment, the plant belongs to the genus *Papaver,* preferably to a species selected from *Papaver somniferum, Papaver bracteatum, Papaver cylindricum, Papaver orientale, Papaver setigerum, Papaver pseudo-orientale, Papaver lauricola, Papaver persicum, Papaver caucasium, Papaver carmeli, Papaver arenarium, Papaver argemone, Papaver atlanticum, Papaver commutatum, Papaver dubium, Papaver fugax, Papaver hybridum, Papaver lateritium, Papaver oreophilum, Papaver pavonium, Papaver pilosum, Papaver pyrenaicum,* more preferably, the plant is selected from *Papaver somniferum, Papaver bracteatum* and *Papaver setigerum* which naturally produce morphinan alkaloids.

The transgenic plant, plant part, and/or plant cells of the present invention which have increased morphinan alkaloids production may be used for the production of morphinan alkaloids as described below.

### Method for Producing Morphinan Alkaloids

The present invention provides a method for producing morphinan alkaloids using the transgenic plant, the plant part, the plant cell and the polypeptide of the present invention.

In particular, the present invention provides a method for producing morphinan alkaloids, comprising the steps of:
(a) growing the plant obtainable by the method for producing a plant or a plant part having altered alkaloid production under suitable conditions which permit the expression of the polypeptide of the present invention; and
(b) recovering morphinan alkaloids from the plant or plant part.

The present invention provides an alternative method of producing morphinan alkaloids, comprising the steps of:
(a) culturing the plant cell culture obtainable by the method for producing a plant cell culture having altered alkaloid production under suitable conditions which permit the expression of the polypeptide of the present invention; and
(b) recovering morphinan alkaloids from the plant cell culture.

The present invention provides a further alternative method of producing morphinan alkaloids, comprising the steps of:
(a) contacting a substrate-containing sample with the polypeptide of the present invention under suitable conditions which allow the reduction of salutaridine into salutaridinol; and
(b) recovering morphinan alkaloids.

In one embodiment, the morphinan alkaloid is salutaridinol. In another embodiment, the morphinan alkaloid is morphine.

The polypeptide of the present invention may be in a purified form, a partially purified form, or in the form of a crude protein extract of an expression host cell, or in the form of an expression host cell with a permeabilized membrane. The polypeptide of the present invention may be in a soluble form or as an immobilized polypeptide on a solid support.

The solid support may be polysaccharide derivatives such as cellulose, dextran, agarose, and polyacrylamide gel or silica, proteins, carbon, polystyrenes, polyacrylates, maleic anhydride based copolymers, polypeptides, vinyl and allyl polymers, and polyamides.

The polypeptide may be immobilized onto the solid support via polypeptide-antibody interaction, tag-antibody interaction, tag-specific binding partner interaction, or any other specific or non-specific, covalent or non-covalent interaction known in the art including gel entrapment.

The term "substrate-containing sample" refers to any sample that contains the substrate (e.g., salutaridine) for salutaridine reductase or reagents which are necessary and sufficient to generate the substrate for salutaridine reductase. Reagents which are necessary and sufficient to generate the substrate for salutaridine reductase include any combination of enyzmes and their respective substrates, which under appropriate conditions, can yield salutaridine. Such reagents will typically include enyzmes which are upstream to salutaridine reductase in the morphine biosynthesis pathway (Figure 1) and their respective substrates. For example, a substrate-containing sample of the present invention may contain P450 oxygenase salutaridine synthase and its substrate (R)-reticuline.

The substrate-containing sample of the present invention also contains additional factors which are required for the reductase activity of salutaridine reductase. Such addition factors include cofactors (e.g., NADPH). Furthermore, the substrate-containing sample of the present invention has the appriorate pH and ionic strength which support the reductase activity of salutaridine reductase.

An examplary substrate-containing sample of the present invention contains 150mM potassium phosphate, pH 6, 500 µM NADPH, and 100 µM salutaridine.

The morphinan alkaloids, in particular, morphine, produced by the method of the present invention may be used for the preparation of a medicament for reducing and/or eliminating pain and/or reducing and/or suppressing coughing.

### Use

The present invention provides the use of the nucleic acid or the vector of the present invention for modulating alkaloid production in a plant, a plant part, or a plant cell as described previously.

The present invention also provides the use of the host cell, the transgenic plant or a part thereof, or the polypeptide of the present invention for producing morphinan alkaloids as described above.

The present invention further provides the use of the transgenic plant or a part thereof of the present invention for the preparation of a composition for reducing and/or eliminating pain and/or reducing and/or suppressing coughing.

The present invention is further illustrated in the following examples.

### EXAMPLES

### Example 1. Materials and Methods

The seeds of all the *Papaver* plants were obtained either from the seed stock collection of the Department of Natural Product Biotechnology of the Leibniz Institute of Plant Biochemistry, Halle, Germany or were the kind gift of Tasmanian Alkaloids Pty Ltd, Westbury, Tasmania. *P. somniferum* L. plants used for the sequencing project as well as the *Papaver* plants used for alkaloid and expression analysis were grown outdoors in Saxony-Anhalt. Peppermint *(Mentha)* plants were obtained from a local market. *Arabidopsis thaliana* ecotype Columbia were cultivated in controlled chambers (Percival, CLF) at 70% relative humidity under short-day conditions of 8 h light, 210 µE /m² /s.

Purified mRNA isolated from *P. somniferum* stems was reverse transcribed and used for construction of a directionally cloned cDNAs library following the instructions for the Superscript Lambda System for cDNA synthesis and □ cloning (Gibco). The thus generated λZipLox cDNA library was subjected to library mass excision resulting in cDNAs directionally cloned into the plasmid pZL1 propagated in the *E.coli* strain DH10B. Randomly chosen bacterial colonies were picked and grown overnight for plasmid purification. The plasmids were digested with Pstl and *Hind*III and insert length was monitored by agarose gel electrophoresis. All plasmids showing inserts at least 500 bp in length were sequenced using the ABI Prism BigDye Terminator Cycle Sequencing Ready Reaction Kit (Applied Biosystems) with the T7 primer. Sequencing reactions were run after excess dye removal on the ABI Prism 310 Sequencer. After trimming for vector, the sequences were clustered and assembled using the SeqManll (DNASTAR Inc.) application using a minimum match size of 20 and a minimum match percentage of 85.

cDNAs for spotting were amplified by PCR using vector specific primers adjacent to the multiple cloning site, purified by filtration through NucleoFast 96 PCR-plates (Macherey-Nagel), and concentrated to at least 100 ng /µL. cDNAs were spotted in quadruplicate with a 384 pin tool on Biodyne B membranes (PALL Corporation) using the Microgrid II spotter (BioRobotics). After spotting, the cDNAs were denatured by incubating the membranes on 0.5 N NaOH / 1.5 M NaCl followed by neutralization on 1 M Tris-HCl pH 7.5 / 1.5 M NaCl. The arrayed DNA was immobilized via UV crosslinking at 120 mjoules / cm using a Stratalinker 1800 (Stratagene).

Total RNA was taken as a template for reverse transcription using oligo-dT primers. After purification by gel filtration through ProbeQuant G-50 Micro columns (Amersham Biosciences) the cDNAs were labelled with [α-³³P]-dATP using the Megaprime DNA Labelling System (Amersham Biosciences). The membranes were incubated in prehybridization solution (5 × SSC, 0.1 % SDS, 5 × Dehnhardt solution and 125 µg / mL denatured salmon sperm DNA) for a minimum of 4 h at 65 °C and overnight hybridisation at 65°C was performed by addition of the labelled cDNAs to the prehybridization solution. The filters were washed 3 times for 15 min with 2 x SSC, 0.1 % SDS at 65°C and exposed to Storage Phosphor Screens. The signals were recorded with a STORM 860 Gel and Blot Imaging System (Amersham Biosciences) and spot intensities and background subtraction were determined using the AIDA array software (Raytest). The background subtracted data of the spot intensities for each experiment were exported in the Scientific Graphing and Analysis Software package Origin V7.5 which permits automated handling of the data analysis and visualization by programming. The analysis is similar to the one described previously (Sreenivasulu et al. 2004), emphasizing different expressions irrespective of absolute signal intensities. However, both median centering procedures extend to all four replicate spots and the averaging is carried out after this procedure in order to prove the statistical significance of the resulting signal intensities. The median centering of arrays (Eisen et al. 1998) sets the median of the logarithmically scaled (log₂) intensity distribution of all genes within an experiment to zero allowing a comparison between distinct experiments. This is followed by a median centering of these array-centered data for each (inclusive replicates) across the experiments. In this way, the previous centering is slightly displaced again. Thus, both median centerings are iteratively repeated until the displacement is reduced to a value of <0.001, which was established after six iterations in this work. In order to prove the significance of the different expressions (x) the averaging were performed as last step, i.e. after the iterative median centering procedure. The results differ only marginally compared with the initial averaging. Thus, the significance (t-test) of each gene expression in all experiments can be determined. Generally, the occurrence of gene expressions with maximum twofold, fourfold, eightfold, or higher over- / underexpression obeys an exponential decaying function. In our analysis the corresponding relative portions are 84.9%, 12.6%, 2.3%, 0.29% of all gene expressions, respectively. Of course, the relative fraction of significant gene expression depends on the elected significance level (α). At α=0.05 this fraction increased from 62% for expressions nearby zero (|x|≤ 1) to ≈95% at higher expressions (|x|>1). The gene expressions of the cluster shown in Fig. 3 reflect the general result with 76.6% significant gene expressions. Complete linkage hierarchical clustering with uncentered Pearson correlation metric was performed using the program Cluster, version 3.0 (developed by Michael Eisen, Stanford University and Michiel de Hoon, University of Tokyo). The TreeView program (version 1.0.10) was used for visualization of the clustering results.

Plant material was ground with a mortar and pestle under liquid nitrogen to a fine powder and the alkaloids extracted with 80% (v/v) ethanol at 4°C for 30 min. After centrifugation at 14,000 g for 10 min and evaporation of the solvent in a Speedvac concentrator, the residue was taken up in methanol.

HPLC analysis for was performed using an LC 1100 series Agilent system equipped with a Lichrospher 60 RP-select B column (250 x 4 mm, 5 µm; Merck) and a solvent system consisting of A: CH₃CN-H₂O (2:98; v/v), solvent B: CH₃CN-H₂O (98:2; v/v) each with 0.02 % (v/v) phosphoric acid. The gradient was from 0%B to 60%B in 25 min with a hold for 5 min followed by an increase to 100% B in 2 min and hold for 3 min at a flow rate of 1 mL / min. The detection wavelength was set to 210 nm. For the analysis of the enzyme assays containing salutaridine or salutaridinol, the gradient was from 25%B to 30%B in 6 min.

ESI-MS measurements and LC separations were carried out on a Mariner TOF mass spectrometer (Applied Biosystems) equipped with a Turbulon Spray source (PE-Sciex) using an LC1100 series Agilent system adapted to flow rates of 0.2 mL / min. Samples were injected on a Superspher 60 RP-Select B column (125 x 2 mm, 5 µm; Merck). The following LC conditions were used: solvent A: CH₃CN-H₂O (2:98; v/v) and solvent B: CH₃CN-H₂O (98:2; v/v), 0.2 % (v/v) formic acid in both solvents. The gradient increased from 0% to 46% B in 25 min, to 90% in 1 min and was held at 90% for 7 min, post time was 5 min. The TOF mass spectrometer was operated in the positive ion mode, with nebulizer gas (N₂) flow of 0.5 L / min, curtain gas (N₂) flow of 1.5 L / min and heater gas flow of (N₂) 7 L / min. The spray tip potential of the ion source was 5.5 kV, the heater and quadrupole temperature were 320°C and 140°C, respectively, the nozzle potential was set to 200 V, and the detector voltage to 1.56 kV. The other settings varied depending on tuning.

The high resolution positive ion ESI mass spectra of the *Papaver* extracts were obtained from a Bruker Apex III Fourier transform ion cyclotron resonance (FT-ICR) mass spectrometer (Bruker Daltonics, Billerica, USA) equipped with an Infinity^{™} cell, a 7.0 Tesla superconducting magnet (Bruker, Karlsruhe, Germany), an RF-only hexapole ion guide and an external electrospray ion source (Agilent, off axis spray, voltages: endplate, -3.700V; capillary, -4.200V; capillary exit, 100V; skimmer 1, 15.0 V; skimmer 2, 10.0 V). Nitrogen was used as drying gas at 150°C. The sample solutions were introduced continuously via a syringe pump with a flow rate of 120 µL / h. All data were acquired with 512 k data points and zero filled to 2048 k by averaging 32 scans.

The LC-ESI-MS and LC-ESI-MS/MS spectra of the [M+H]⁺ ions of alkaloids were obtained from a Finnigan MAT TSQ 7000 system (electrospray voltage 4.5 kV, sheath gas: nitrogen; capillary temperature: 220 °C; collision gas: argon; collision pressure: ca. 1.8 x 10⁻³ Torr; collision energy depended on structure). The MS system is coupled with a Surveyor micro-HPLC (Thermofinnigan) and equipped with a Ultrasep ES RP18E-column (5 µm, 1x100 mm, SepServ). For the HPLC a gradient system was used starting from H₂O:CH₃C 85:15 (each of them containing 0.2% (v/v) HOAc) to 10:90 within 15 min; flow rate 50 µL / min. All mass spectra are averaged and background subtracted.

### 1.5 GC-MS Analysis

The samples from the menthone and tropinone reductase assays were analysed on a Finnigan TraceGC gas chromatograph coupled to a Finnigan Polaris Q mass spectrometer. Separation was performed on (5%-diphenyl)-dimethylpolysiloxane column (Restek, Bad Homburg, Germany) of 15 m x 0.25 mm i.d. x 0.25 µm film thickness. Helium was the carrier flow gas (flow rate of 1 mL/min), a splitless injection (injection volume of 1µl, injection temperature 220°C) was used, and temperature gradients of 2°C / min from 40°C (1 min hold) to 80°C followed by 100°C / min to 150°C, or of 3°C / min from 40°C (1 min hold) to 100°C followed by 100°C / min to 150°C were applied for menthol or tropinone samples, respectively. Mass spectrometry was performed with an interface temperature of 300°C, ion source temperature of 200°C, and an ionization potential of 70 eV. The compounds were identified by their El-spectra and comparison with a NIST database or compared with authentic standards obtained from Fluka.

The missing 5'-end of cDNA clone 16B1 was amplified according to the SMART^{™} cDNA amplification kit (Clontech, Heidelberg, Germany) using the following gene-specific primers: 16B1r1 5'-ACCTTCTTCGGCAG-3' for reverse transcription of mRNA isolated from *P. somniferum* stems, and 16B1 r2 5'-CAGCTCCGAAACTAGGCCACCCATTTGTTTCG ATC-3' for PCR (30 s 94°C, 10 cycles 30 s 94°C, 30 s 70°C, 2 min 72°C with decreasing annealing temperature by 1°C per cycle, followed by 25 cycles 30 s 94°C, 30 s 60°C, 2 min 72°C and a final elongation for 10 min at 72°C). The resulting band of 900 bp was ligated into pGEMTeasy (Promega) and sequenced. The entire open reading frame for over-expression was obtained by RT-PCR using PfuUltra^{™} Hotstart DNA Polymerase (Stratagene) and the primers 16B1-5-2: 5'-ATGCCTGAAACATGTCC-3' and 16B1-3-1: 5'-ATAACTCAAAAT GCAGATAGTTCTG-3'. The PCR conditions were as described above at a constant annealing temperature of 54°C. The resulting fragment of approximately 1000 bp was ligated into pBluescriptIISK and sequenced.

The open reading frame of the cDNA 16B1 was excised from the pBluescriptIISK vector with *Kpn*I and *Pst*I and ligated into the expression vector pQE-30 (Qiagen). For over-expression, the recombinant plasmid was transformed into *E. coli* SG13009. Single isolated bacterial colonies from freshly streaked plates (grown on Luria-Bertani (LB) agar medium containing 50 µg/mL ampicillin and 50 µg/mL kanamycin) were used to inoculate 2 mL liquid cultures (LB medium containing 50 µg/mL ampicillin and 50 µg/mL kanamycin), which were grown overnight at 37°C. An aliquot (100 µL) of these cultures was used to inoculate 50 mL liquid cultures. At a cell density of 0.5 OD₆₀₀, recombinant protein expression was induced with 1 mM isopropyl-□-D-thiogalactopyranoside. After incubation at 37°C for 4 hours, cells were harvested and sonicated in extraction buffer (20 mM Tris-HCl pH 7.5, 100 mM KCI, 10% (v/v) glycerol, 20 mM □-mercaptoethanol, 10 µg / mL lysozyme). After removal of the cell debris by centrifugation, the supernatant was loaded onto a cobalt affinity column (Talon, Clontech), followed by washing with extraction buffer without lysozyme. The recombinant protein was eluted by a stepwise increase in the imidazole concentration from 10 to 30 mM.

### 1.8 Cloning and Expression of a Peppermint cDNA encoding (-)-Menthone Reductase and of the cDNA of Arabidopsis Gene At3g61220

The mRNA from *Arabidosis thaliana* and from peppermint leaves was reverse transcribed with an oligo-dT primer. The open reading frame of (-)-menthone reductase was amplified from peppermint leaf cDNA using primers MMNR-5-1: 5'- CACCATGGGAGATGAAGTAGT CGTC-3' and MMNR-3-1: 5'-ATACAAGCAGAACGCTTCGTCTC-3' as deduced from the MpMNR sequence (accession number AY288137). The open reading from the Arabidopsis homologue of 16B1 was amplified by RT-PCR with primers: Ath16B1-5-1 5'-CACCATGGCAGAGGAAACTCC-3' and Ath16B1-3-1: 5'-GAATTCTGAAACTTGCTT GCGAC-3' as deduced from sequence deposited in the database under the accession number AY091305. Both cDNAs were amplified using PfuUltra^{™} Hotstart DNA Polymerase (Stratagene) under the following PCR conditions: 30 s 94°C, 25 cycles 30 s 94°C, 30 s 55°C, 2 min 72°C and a final elongation for 10 min at 72°C. The resulting fragments were blunt end ligated into pQE30, and the recombinant plasmids were transformed into *E. coli* SG13009. Expression of the cDNAs was performed as described for *P. somniferum* 16B1. The cells were harvested and sonicated in extraction buffer (50 mM MOPSO pH 7, 10% (v/v) glycerol, 10% (w/v) sorbitol, 10 mM □-mercaptoethanol, 10 µg / mL lysozyme). For purification of the recombinant proteins, the bacterial crude extract was adjusted to an imidazole concentration of 10 mM and loaded onto spin columns from the ProPur Mini Kit (Nunc). Purification of the proteins was achieved by an increase in imidazole concentration from 50 to 100 mM.

### 1.9 Enzyme Assays and Enzyme Characterization

The enzyme assay reaction mixture for the reduction of substrates consisted of 150 mM potassium phosphate pH 6, 500 µM NADPH, 100 µM salutaridine, codeinone or tropinone, and up to 100 µL protein extract in a total volume of 200 µL. For the reverse reaction, 150 mM glycine buffer pH 9.5, 500 µM NADP and 100 µM salutaridinol or codeine were used. The enzyme assays for the reduction of 1,2-dehydroreticulinium ion and 1,2-nordehydroreticuline were performed in 300 mM glycine buffer pH 8.5 with 100 µM substrate. After incubation at 30°C, the enzymatic reaction was terminated by the addition of 200 µL 1 M NaHCO₃ and products extracted with 500 µL ethylacetate. The organic phase was removed *in vacuo,* the residue was taken up in 50 µL methanol and analyzed as described in the analysis of alkaloid section. For enzyme assays containing tropinone, the compounds were extracted with 1 mL chloroform. The (-)-menthone reductase assays contained 40 mM potassium phosphate buffer pH 7, 10 mM β-mercaptoethanol, 100 µM (-)-menthone, 500 µM NADPH and up to 150 µL protein extract in a total volume of 500 µL. After incubation at 30°C, the products were extracted with 1 mL hexane. The reaction products of the incubations with (-)-menthone and tropinone were analysed by GC-MS.

Saturation curves and double reciprocal plots were constructed with the FIG.P program version 2.98 (Biosoft, Cambridge, UK). The influence of pH on enzyme activity was monitored in sodium citrate (pH 4-6), potassium phosphate (pH 6-8), Tris-HCl (pH 7-9), Tricine-NaOH (pH 8.5-9.5) and glycine / NaOH (pH 9-12) buffered solutions. The subunit molecular mass was determined by SDS-PAGE (12% polyacrylamide) according to Lämmli (1974). The native molecular mass was estimated by gel filtration chromatography through a Superdex 75Hiload Prep 16/60 column (Amersham Bioscience) with bovine serum albumin (67kD), ovalbumin (45 kD), and α-chymotrypsinogen (25 kD) as standards. Protein concentrations were measured according to Bradford (1976).

Total RNA was isolated with TRIzol according to the manufacturer's protocol. Northern blot analyses were performed as described by Ausubel (1987). The 16B1 probes were produced by amplifying fragments with the primers 16B1-5-2 and 16B1-3-1 the full-length cDNA from *P. somniferum* or the first strand cDNA from *P. bracteatum* and labelling with [α-³²P]dATP. Hybridization conditions were as described for macroarray hybridization.

### 1.11 Accession Numbers

Sequence data from this article have been deposited with the EMBL/GenBank data libraries under the accession numbers DQ31621 for *P. somniferum* salutaridine reductase and DQ362936 for the Mentha cDNA with (-)-menthone:(+)-neomenthol activity. The sequences from the EST sequencing project can be accessed *via* the homepage of The Floral Genome Project (http://www.floralgenome.org/).

### Example 2. Expressed Sequence Tag Sequencing Project from P. somniferum

In order to obtain additional cDNAs for macroarray analysis, we initiated an EST-sequencing project from *P. somniferum* stem sections two cm in length adjacent to the capsule. This tissue has been previously shown to contain high amounts of benzylisoquinoline alkaloids accompanied by high expression of most of the cDNAs coding for enzymes in benzylisoquinoline biosynthesis isolated thus far (Unterlinner et al., 1999, Huang and Kutchan, 2000, Grothe et al., 2001; Facchini and Park, 2003, Ounaroon et al., 2003). The cDNA library used for sequencing was primed from the 3'-end of the mRNAs and only cDNAs longer than 500bp were sequenced from the 5'-end. In total, 2078 cDNAs were sequenced producing a set of 1152 UniGenes (55.4%) consisting of 865 singletons and 287 assemblies. Each UniGene was submitted to the non-redundant database on the NCBI server using the blastx algorithm. Using an expect value of (E-value) of 10⁻⁹ as threshold, slightly more than 50% of the UniGenes either showed no significant homology (28%) or homology to hypothetical or expressed proteins of unknown function (24%) (Figure 2). Additionally, 1% of the UniGenes and 3% of all sequences coded for proteins of viral origin with the coat protein from the turnip mosaic virus as the most abundant representative.

The remaining UniGenes mainly coded for proteins involved in cell growth, cell division, DNA-and protein synthesis (33%) followed by those implicated in metabolism (25%) and in stress response and redox control (12%). Most of the remaining 30% of the UniGenes coded for proteins related to processes in photosynthesis (7%) and with functions in transport and cellular communication / signal transduction (8% each). Considering all sequences, and not only the UniGene set, a similar distribution into functional categories was observed. The only strong differences were observed for proteins involved in stress response and redox control as well as for major latex proteins (MLP). *MLPs* constitute 1% of the UniGenes, but 5% of all sequences, which is representative of the high abundance of these proteins in laticifers (Nessler and von der Haar, 1990). The two-fold difference in the representation of cDNAs coding for proteins implicated in stress responses and redox control in all sequences compared to the UniGene set is due to the high abundance of a metallothionein, with 134 sequences. 1.8% of the UniGenes could be assigned to secondary metabolism including four enzymes implicated in benzylisoquinoline biosynthesis, TYDC, Cyp80b3, 4'-OMT and COR. Other sequences with potential functions in secondary metabolism include eighteen transcription factors, eight dehydrogenases / reductases, seven methyltransferases, six cytochrome P450-dependent monooxygenases, three oxygenases and two ABC transporters.

### Example 3. Alkaloid Composition of Papaver Species

In a previous analysis aimed at isolating cDNAs that make *P. somniferum* capable of production of morphinan alkaloids, nine other *Papaver* species devoid of this benzylisoquinoline class were compared with three *P. somniferum* varieties (Ziegler et al., 2005). In the current investigation, seven additional *Papaver* species and three more *P. somniferum* varieties were included. The *Papaver* species can be classified into twelve sections according to Preininger (1986), which are based on morphological characteristics. Although there are many reports in the literature about the alkaloid composition of many *Papaver* species, considerable variation can occur depending on the origin of the species, the tissue extracted as well as on the growth conditions. It was, therefore, necessary to analyse the alkaloid composition of each plant species that was later used for expression analysis. A section of the stem about two cm in length adjacent to the base of the capsule was harvested from field-grown plants and one slice of two mm thickness was excised and used for alkaloid extraction and analysis. A major focus of this analysis was to investigate which plants produce alkaloids of the morphinan type. In order to get a first overview, the presence of masses indicative for morphinan alkaloids was examined by ESI-FT-ICRMS. If such masses were present in the plants, HPLC-MS and HPLC-MS-MS analysis were conducted to see whether the respective alkaloid belongs to the morphinan or to another benzylisoquinoline class. In most of the *P. somniferum* varieties masses of [M+H⁺] *m*/*z* 286, *m*/*z* 298, *m*/*z* 300, *m*/*z* 312, *m*/*z* 328 and *m*/*z* 330 could be detected, which could be identified by HPLC-MS as the morphinan alkaloids morphine, codeinone /oripavine, codeine, thebaine, salutaridine /1,2-dehydroreticulinium ion, and salutaridinol, respectively. Only in the *P. somniferum* mutant *top1,* masses for codeine and morphine could not be detected (Table 2) (Millgate et al., 2004). Masses indicative of morphinan alkaloids also occurred in extracts of other *Papaver* species, but only in *P. arenarium,* and *P. bracteatum,* LC-ESI-MS-MS analysis showed characteristic fragments for a morphinan structure. These signals could be identified as the morphinan alkaloids N-demethylcodeine, thebaine and thebaine-N-oxide. The major alkaloids in other *Papaver* species belonged to the scoulerine-derived papaverrubines, protoberberines, protopines and rhoeadines (data not shown).

**Table 2. Alkaloid composition of Papaver**

| species | | [M+H]⁺ *m*/*z* indicative of morphinans^{a} | elemental composition | error (ppm) | morphinan alkaloid |
|---|---|---|---|---|---|
| *P. argemone* | | no | | | no |
| *P. arenarium* | | 286.14388 | [C₁₇H₂₀NO₃]+ | 0.5 | N-demethylcodeine |
| | | 312.15945 | [C₁₉H₂₂NO₃]+ | 0.2 | |
| | | 328.15417 | [C₁₉H₂₂NO₄]+ | 0.5 | |
| *P. commutatum* | | no | | | no |
| *P. bracteatum* | | 312.15948 | [C₁₉H₂₂NO₃]+ | 0.2 | thebaine |
| | | 328.15441 | [C₁₉H₂₂NO₄]+ | 0.2 | thebaine-*N*-oxide |
| *P. orientale* | | 312.15953 | [C₁₉H₂₂NO₃]+ | 0.4 | no |
| | | 328.15447 | [C₁₉H₂₂NO₄]+ | 0.4 | no |
| | | 330.17013 | [C₁₉H₂₄NO₄]+ | 0.5 | no |
| *P.lateritium* | | 300.15947 | [C₁₈H₂₂NO₃]+ | 0.3 | no |
| *P. pyrenaicum* | | 298.14425 | [C₁₈H₂₂NO₃]+ | 1.6 | no |
| | | 300.15980 | [C₁₈H₂₀NO₃]+ | 1.3 | no |
| | | 328.15452 | [C₁₉H₂₂NO₄]+ | 0.6 | no |
| | | 330.17050 | [C₁₉H₂₄NO₄]+ | 1.6 | no |
| *P. atlanticum* | | 300.15890 | [C₁₈H₂₀NO₃]+ | 1.7 | no |
| *P. pilosum* | | 300.15908 | [C₁₈H₂₀NO₃]+ | 1.1 | no |
| | | 328.15389 | [C₁₉H₂₂NO₄]+ | 1.3 | no |
| *P. fugax* | | 298.14303 | [C₁₈H₁₈NO₃]+ | 2.5 | no |
| | | 328.15365 | [C₁₉H₂₂NO₄]+ | 2.1 | no |
| *P. oreophilum* | | 328.15412 | [C₁₉H₂₂NO₄]+ | 0.6 | no |
| *P. hybridum* | | no | | | no |
| *P. glaucum* | | no | | | no |
| *P. somniferum* | | | | | |
| | Paso | all | | | all |
| | Nopa | all | | | all |
| | Nosca | all | | | all |
| | Papa | all | | | all |
| | Fool Ori | all | | | all |
| | *top 1*^{c} | all but | | | all but codeine, |
| | | 300.15942, | | | morphine, codeinone |
| | | 286.14376 | | | |

| | | | | | |
|---|---|---|---|---|---|
| ^{a} masses indicative for morphinan alkaloids are: 286.14376 (morphine), 298.14376 (oripavine, codeinone, 300.15942 (codeine), 312.15942 (thebaine), 328.15433 (salutaridine, 1,2-dehydro- | | | | | |

### Example 4. Gene Expression Analysis of Papaver Species

In order to correlate the alkaloid profile with the gene expression for each individual plant, the RNA was extracted from the remainder of the stems, reverse transcribed, labelled with [α-³³P]dATP and hybridized to the macroarrays. The macroarrays contained the 1152 UniGenes from the EST-project as well as the 849 UniGenes described in Ziegler et al. (2005) as PCR fragments, spotted in quadruplicate. Additionally, eight cDNAs coding for enzymes involved in benzylisoquinoline biosynthesis, which had been already isolated from *P. somniferum,* were included. In total, 54 individual plants were analysed, among them 23 *P. somniferum* plants from six varieties (5x PaSo, 2x Nopa, 5x Nosca, 3x Papa, 4x Fool Ori, 4x *top1)* and 31 plants from other species (4x *P. bracteatum,* 3x each *P. glaucum* and *P. orientale,* 2x each *P. arenarium, P. argemone, P. atlanticum, P. commutatum, P. fugax, P. hybridum, P. lateritium, P. pilosum, P, pyrenaicum,* as well as one plant each from *P. dubium, P. oreophilum, P. pavonium).* Hierarchical clustering of the expression data exhibited six clusters showing enhanced expression of cDNAs in *P. somniferum* varieties compared to other *Papaver* species. Interestingly, seven out of the eight cDNAs involved in benzylisoquinoline biosynthesis cluster together (Figure 3), such as cDNAs for the early pathway up to (S)-reticuline *(6-OMT, Cyp80B1, 4'-OMT)* as well as cDNAs involved in the morphinan-specific branch of the pathway *(SalAT,* COR). In total, 69 cDNAs are present in these six clusters, of which 62% showed no homology to the database or homology to uncharacterised genes. Eight cDNAs code for proteins presumably involved in secondary metabolism, among them six P450 dependent monooxygenases, of which four are present in the cluster shown in Figure 3. The whole median centered dataset can be viewed in the Supplemental Table 1, which can be imported in to the cluster program for further analysis. The intent of this study was to isolate and characterize cDNAs putatively involved in morphinan alkaloid accumulation with a major focus on those coding for enzymes presumably catalysing reactions in benzylisoquinoline biosynthesis. Since most of the cDNAs that are already known to be involved in alkaloid biosynthesis show very similar and higher expression in morphinan producing *P. somniferum* plants compared to other *Papaver* species, we first focussed on that cluster in order to choose ESTs for further characterization. The clone 16B1 showed higher expression in *P. somniferum* plants compared to other plants and this expression is tightly correlated to the expression of other cDNAs involved in benzylisoquinoline biosynthesis such as *4'-OMT, COR* or *6-OMT.* This clone exhibited the highest amino acid sequence similarity to a putative short chain dehydrogenase / reductase from *Arabidopsis thaliana.* Members of this enzyme family have already been shown to catalyse reactions in secondary metabolism, for example the stereospecific reduction of tropinone to tropine in tropane alkaloid metabolism (Nakajima et al., 1999). In morphine biosynthesis, there are three reductive candidate steps, the reduction of 1,2-dehydroreticulinium ion to (R)-reticuline, of salutaridine to salutaridinol, and from codeinone to codeine, respectively. Because of its expression profile and its homology to enzymes catalysing reduction in secondary metabolite biosynthesis, we chose this clone for further characterization to investigate a putative function in benzylisoquinoline metabolism.

### Example 5. Analysis of Clone 16B1 and its Full Length Sequence

The clone 16B1 comprised 750 bp and showed the highest homology to a putative SDR from *Arabidopsis thaliana* with an amino acid identity of 57% covering amino acid positions 120 to the C-terminus. Thus, roughly 400 nucleotides from the N-terminal sequence were missing in the *Papaver* EST, which were amplified by 5-RACE from *P. somniferum* RNA. The assembly of the 5'-RACE sequence with the EST yielded a cDNA of 1,326 bp with an open reading frame of 936 bp coding for a protein of 311 amino acids with a molecular mass of 34.1 kD and an isoelectric point of 4.69. In accordance with the macroarray data, northern blot analysis using the full length sequence of clone 16B1 as a probe showed the highest expression in morphinan alkaloid containing *P. somniferum* varieties, *P. arenarium* and *P. bracteatum,* whereas the mRNA level in other *Papaver* species was very low or not detectable (Figure 4). To investigate the possibility that differences in signal intensities could be due to low sequence homology between the *P. somniferum-*specific probe and the respective sequence from the other *Papaver* species, the homologous cDNA was amplified and sequenced from *P. bracteatum.* The sequence showed 96% identity on the nucleotide level to the *P. somniferum* cDNA. Hybridization of the same northern blot with labelled *P. bracteatum* cDNA as probe showed a similar result as with the *P. somniferum* probe, thus confirming the differential expression of this gene between species rather than differences in hybridization kinetics.

The full-length sequence of 16B1 exhibits several motifs characteristic for the large family of short chain dehydrogenasae/ reductases such as the GxxxGxG segment characteristic of the coenzyme binding fold in dehydrogenases or the catalytic YxxxK motif (Figure 5, Jörnvall et al., 1995). Database search revealed the highest homology to an uncharacterised SDR from *Arabidopsis thaliana* (At3g61220; 55% amino acid identity), and to three SDRs acting in monoterpene metabolism from *Mentha x piperita,* (-)-menthone:(+)-(3S)-neomenthol reductase (MpMNR), (-)-isopiperitenone reductase (MpISPR) and (-)-menthone:(-)-(3R)-menthol reductase (MpMMR) with more than 45% identity (Figure 5; Ringer et al., 2003; Davis et al., 2005).

### Example 6. Purification and Functional Characterization of the Recombinant Enzyme

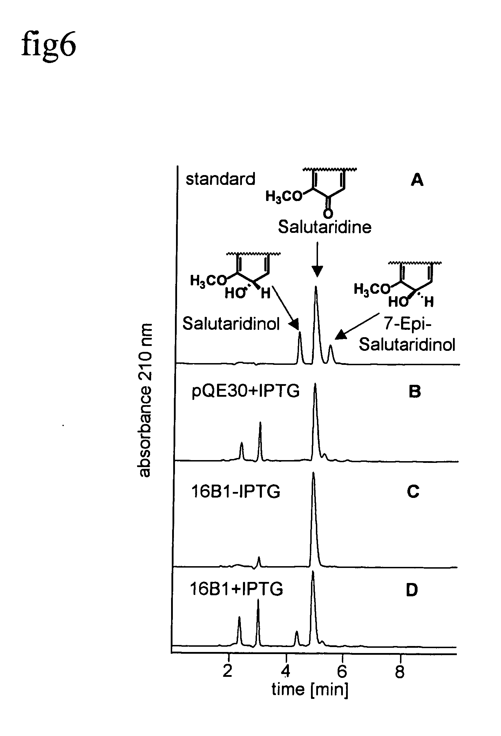

The full-length sequence was cloned into the expression vector pQE30 containing six-histidine N-terminal extension and over-expressed in *E.coli* strain SG13009. The recombinant protein had a relative molecular mass of 40 kD as determined by SDS-PAGE, which compared favourably with the calculated mass of 37.3 kD deduced from the translation of the cDNA. The native molecular mass between 47.1 kD and 50.1 kD determined by gel filtration on a calibrated Superdex TM75 column revealed the protein to be a monomer, but with an increased mass compared to the result obtained from SDS-PAGE. Based on the sequence homology to SDRs, pathway intermediates that undergo a reduction during morphine biosynthesis were chosen as substrates. Since COR had already been cloned and the recombinant protein proven to be highly specific (Unterlinner et al., 1999), we preferably investigated the reduction from 1,2-dehydroreticulinium ion to (R)-reticuline and the reduction from salutaridine to salutaridinol. After incubation with the recombinant 16B1, conversion of 1,2-dehydroreticulinium ion to reticuline could not be observed after HPLC and HPLC-MS analysis. With salutaridine as substrate, a peak at the same retention time and the same UV-spectrum as salutaridinol could be observed, whereas no signal for the stereoisomer 7-epi-salutaridinol could be detected (Figure 6D). Control incubations with extracts from bacteria that had not been induced or that had been induced but contained empty vector did not show any conversion of salutaridine (Figure 6B,C). Additionally, only the LC-MS analysis of the incubation with the recombinant 16B1 protein showed the [M+H]⁺ ion of salutaridinol (*m*/*z* 330) in addition to the [M+H]⁺ ion of the substrate salutaridine *(m*/*z* 328). Furthermore, characteristic fragments of salutaridinol at *m*/*z* 58 ([CH₃CHNHCH₃]⁺, ethylmethylammonium ion representative for a morphinan structure), *m*/*z* 181 [M+H-CH₂CHNHCH₃-2MeOH-CO]⁺, as well as *m*/*z* 267 [M+H-CH₃NH₂-MeOH]⁺ and its further loss of CO *(m*/*z* 239) were detected by LC-MS-MS (Raith et al., 2003).

The recombinant protein exhibited the highest activity at a temperature between 30°C and 35°C with half maxima at 20°C and 40°C. The pH-dependence for the conversion of salutaridine to salutaridinol by the recombinant protein revealed a pH-optimum between pH 5.5 and 6.0 with a sharp increase starting from pH 4.7 and a slower decrease with half maximal activity at pH 7.5. At pH 9 no activity could be detected. A partially purified protein from *P. somniferum* responsible for the reduction of salutaridine had been shown to catalyze the reverse reaction from salutaridinol to salutaridine in the presence of NADP and at higher pH (Gerardy and Zenk, 1993). The incubation of the recombinant 16B1 protein with salutaridinol and NADP at pH 9.5 and analysis of the reaction products by HPLC and HPLC-MS revealed a mass signal for salutaridine ([M+H]⁺ =m/z 328) in addition to salutaridinol ([M+H]⁺ =m/z 330), showing that the protein is capable to catalyze the reverse reaction. The turnover rate of the recombinant protein for the reverse reaction was maximal at pH 9.5 with a sharp decline to zero activity at pH 10. Only 20% of the maximum activity for the reverse reaction could be observed at the optimum pH for the forward reaction at pH 6. At optimal pH conditions and the same cofactor concentration, the reverse reaction exhibited an almost two-fold higher reaction velocity than the forward reaction, whereas the *K*ₘ values for the substrates were in a similar range with 30.9 µM for salutaridine and 23 µM for salutaridinol (Table 3). The kinetic data showed a two-fold stronger affinity of the enzyme for NADPH in the forward reaction compared with NADP in the reverse reaction, whereas the catalytic efficiency for the substrates was higher for the reverse reaction at a constant cofactor concentration of 500 µM. The recombinant enzyme strongly preferred the phosphorylated cofactor with 20% of the activity when NADH was used instead of NADPH in the forward reaction and 5% of the activity in the reverse reaction when NADP was substituted with NAD.

**Table 3. Kinetic parameters of salutaridine reductase for the forward and reverse reaction and the cofactors**

| Substrate | Kₘcofactor (µM) | Vₘₐₓ cofactor (pkat / mg) | Kₘ substrate (µM) | Vₘₐₓ substrate (pkat / mg) | Vmax/Km |
|---|---|---|---|---|---|
| Salutaridine | 80 ± 9^{a} | 9.4 ± 0.3^{a} | 30.9 ± 13.2` | 5.8 ± 1.3^{c} | 0.18 |
| Salutaridinol | 198 ± 56^{b} | 11.6 ± 1.5^{b} | 23.0 ± 6.9^{d} | 10.6 ± 0.8^{d} | 0.46 |

| | | | | | |
|---|---|---|---|---|---|
| ^{a} NADPH as cofactor, salutaridine concentration 100 µM. ^{b} NADP as cofactor, salutaridinol concentration 100 µM. ^{c} NADPH concentration 500 µM. ^{d} NADP concentration 500 µM. | | | | | |

### Example 7. Substrate Specificity of the Recombinant Protein

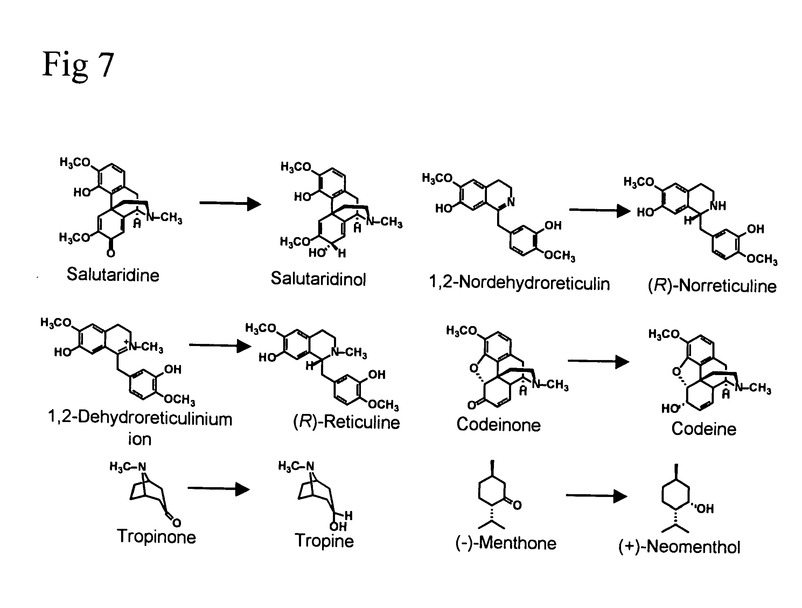

Various benzylisoquinolines were tested as substrate and the reaction products were analyzed by LC-MS (Figure 7). No reduction of the N-C-1 double bond of 1,2-dehydroreticulinium ion to reticuline was detected. Also, norreticuline formation was not observed with its N-demethylated derivative nordehydroreticuline. Using the penultimate morphine precursor codeinone as keto substrate, no conversion to codeine could be observed. Similarly, the reverse reaction with codeine as substrate also showed no generation of codeinone. Since SDR enzymes had been shown to catalyze the stereospecific reduction of tropinone in tropane alkaloid biosynthesis, we included this compound in the investigation. Neither tropine nor pseudo-tropine formation could be detected by GC-MS after the incubation of tropinone with the recombinant enzyme.

The 16B1 protein showed the highest similarity to an SDR of unknown function from *Arabidopsis thaliana* followed by SDRs acting in the biosynthesis of menthol from *Mentha* x *piperita.* This prompted an examination of the possibility whether the recombinant protein accepts monoterpenes as substrates. With menthone, no conversion to neomenthol or menthol could be detected by GC-MS. Even when the same extraction and assay conditions described by Ringer et al. (2003) were used, no activity could be detected. Additionally, it was of interest to test whether the homologous proteins could catalyze the reduction of salutaridine. The open reading frame of the Arabidopsis homolog of the (-)-menthone:(+)-neomenthol reductase from *Arabidopsis thaliana* and from a peppermint variety obtained from a local market were therefore amplified from the corresponding RNA. The deduced amino acid sequence of the Arabidopsis protein was identical to the database entry, whereas the MNR from peppermint showed an amino acid sequence identity of 75% to the (-)-menthone:(+)-neomenthol reductase and of 67% to the (-)-menthone:(-)-menthol reductase of *Mentha x piperita.* After overexpression and purification, both, the Arabidopsis and the peppermint proteins catalyzed the conversion of (-)-menthone to (+)-neomenthol (95%) and (-)-menthol (5%). However, after purification to homogeneity, the specific activity of the Arabidopsis protein was 20-fold lower compared to the protein from peppermint. When the same enzyme preparations were incubated with salutaridine, conversion to salutaridinol was not observed, independent of the extraction and assay conditions.

### REFERENCES

Aharoni, A., Giri, A.P., Verstappen, F.W., Bertea, C.M., Sevenier, R., Sun, Z., Jongsma, M.A., Schwab, W., and Bouwmeester, H.J. (2004) Gain and loss of fruit flavor compounds produced by wild and cultivated strawberry species. Plant Cell 16, 3110-3131.
Ausubel, F.M. (1987). Current protocols in molecular biology. New York Green Publications, Associates and Wiley-Interscience, New York.
Bomati, E.K., Austin, M.B., Bowman, M.E., Dixon, R.A., and Noel, J.P. (2005) Structural elucidation of chalcone reductase and implications for deoxychalcone biosynthesis. J. Biol. Chem. 280, 30496-30503.
Bradford, M.M. (1976). A rapid and sensitive method for the quantitation of microgram quantities of protein utilizing the principle of protein-dye binding. Anal. Biochem. 72, 248-254.
Chen, F., Tholl, D., D'Auria, J.C., Farooq, A., Pichersky, E., and Gershenzon, J. (2003). Biosynthesis and emission of terpenoid volatiles from Arabidopsis flowers. Plant Cell 15, 481-94.
Choi, K.B., Morishige, T., Shitan, N., Yazaki, K., and Sato, F. (2002). Molecular cloning and characterization of coclaurine N-methyltransferase from cultured cells of Coptis japonica. J. Biol. Chem. 277, 830-835.
Davis, E.M., Ringer, K.L., McConkey, M.E., and Croteau, R. (2005). Monoterpene metabolism. Cloning, expression, and characterization of menthone reductases from peppermint. Plant Physiol. 137, 873-881.
De-Eknamkul, W., and Zenk, M.H. (1992). Purification and properties of 1,2-dehydroreticuline reductase from Papaver somniferum seedlings. Phytochemisty 31, 813-821.
Dittrich, H., and Kutchan, T.M. (1991). Molecular cloning, expression and induction of berberine bridge enzyme, an enzyme essential to the formation of benzophenanthridine alkaloids in the response of plants to pathogenic attack. Proc. Natl. Acad. Sci. USA 88, 9969-9973
Eisen, M.B., Spellman, P.T., Brown, P.O., and Botstein, D. (1998). Cluster analysis and display of genome-wide expression patterns. Proc. Natl. Acad. Sci. USA 88, 9969-9973.
Facchini, P.J., and De Luca, V. (1994). Differential and tissue-specific expression of a gene family for tyrosine / dopa decarboxylase in opium poppy. J. Biol. Chem. 269, 26684-26690.
Facchini, P.J., and Park, S.U. (2003). Developmental and inducible accumulation of gene transcripts involved in alkaloid biosynthesis in opium poppy. Phytochemistry 64, 177-186.
Filling, C., Berndt, K.D., Benach, J., Knapp, S., Prozorovski, T., Nordling, E., Ladenstein, R., Jörnvall, H., and Oppermann, U. (2002). Critical residues for structure and catalysis in short-chain dehydrogenases/reductases. J. Biol. Chem. 277, 25677-25684.
Frick, S., and Kutchan, T.M. (1999). Molecular cloning and functional expression of O-methyltransferases common to isoquinoline alkaloid and phenylpropanoid biosynthesis. Plant J. 17, 329-339.
Fridman, E., and Pichersky, E. (2005). Metabolomics, genomics, proteomics, and the identification of enzymes and their substrates and products. Curr. Opin. Plant. Biol. 8, 242-248.
Fridman, E., Wang, J., Iijima, Y., Froehlich, J.E., Gang, D.R., Ohlrogge, J., and Pichersky, E. (2005). Metabolic, genomic, and biochemical analyses of glandular trichomes from the wild tomato species Lycopersicon hirsutum identify a key enzyme in the biosynthesis of methylketones. Plant Cell 17, 1252-1267.
Gang, D.R., Lavid, N., Zubieta, C., Chen, F., Beuerle, T., Lewinsohn, E., Noel, J.P., and Pichersky, E. (2002). Characterization of phenylpropene O-methyltransferases from sweet basil: facile change of substrate specificity and convergent evolution within a plant O-methyltransferase family. Plant Cell 14, 505-519.
Gerardy, R., and Zenk, M.H. (1992). Formation of satutaridine from (R)-reticuline by a membrane-bound cytochrome P-450 enzyme from Papaver somniferum. Phytochemistry 32, 79-86.
Gerardy, R., and Zenk, M.H. (1993). Purification and characterization of salutaridine: NADPH 7-oxidoreductase from Papaver somniferum. Phytochemistry 34, 125-132.
Ghosh, D., Sawicki, M., Pletnev, V., Erman, M., Ohno, S., Nakajin, S., and Duax, W.L. (2001). Porcine carbonyl reductase. structural basis for a functional monomer in short chain dehydrogenases/reductases. J. Biol. Chem. 276, 18457-18463.
Grothe, T., Lenz, R., and Kutchan, T.M. (2001). Molecular characterization of the salutaridinol 7-O-acetyltransferase involved in morphine biosynthesis in opium poppy Papaver somniferum. J. Biol. Chem. 276, 30717-30723.
Guterman, I., Shalit, M., Menda, N., Piestun, D., Dafny-Yelin, M., Shalev, G., Bar, E., Davydov, O., Ovadis, M., Emanuel, M., Wang, J., Adam, Z., Pichersky, E., Lewinsohn, E., Zamir, D., Vainstein, A., and Weiss, D. (2002) Rose scent: genomics approach to discovering novel floral fragrance-related genes. Plant Cell 14, 2325-2338.
Hirata, K., Poeaknapo, C., Schmidt, J., and Zenk, M.H. (2004). 1,2-Dehydroreticuline synthase, the branch point enzyme opening the morphinan biosynthetic pathway. Phytochemistry. 65,1039-46.
Huang, F.C., and Kutchan, T.M. (2000). Distribution of morphinan and benzophenanthridine alkaloid gene transcript accumulation in the opium poppy Papaver somniferum. Phytochemistry 53, 555-564.
Iijima, Y., Davidovich-Rikanati, R., Fridman, E., Gang, D.R., Bar, E., Lewinsohn, E., and Pichersky, E. (2004). The biochemical and molecular basis for the divergent patterns in the biosynthesis of terpenes and phenylpropenes in the peltate glands of three cultivars of basil. Plant Physiol. 136, 3724-3736.
Ikezawa, N., Tanaka, M., Nagayoshi, M., Shinkyo, R., Sakaki, T., Inouye, K., and Sato, F. (2003). Molecular cloning and characterization of CYP719, a methylenedioxy bridge-forming enzyme that belongs to a novel P450 family, from cultured Coptis japonica cells. J. Biol. Chem. 278, 38557-38565.
Jörnvall, H., Höög, J.O., and Persson, B. (1999). SDR and MDR: completed genome sequences show these protein families to be large, of old origin, and of complex nature. FEBS Lett. 445, 261-264.
Jörnvall, H., Persson, B., Krook, M., Atrian, S., Gonzàlez-Duarte, R., Jeffery, J., and Ghosh, D. (1995). Short-chain dehydrogenases/reductases (SDR). Biochemistry. 34, 6003-6013.
Kutchan, T.M. (1998).Molecular genetics of plant alkaloid biosynthesis. In The Alkaloids Vol. 50, G. Cordell, ed (San Diego, Academic Press) pp. 257-316.
Kutchan, T.M., Frick, S., and Weid, M. (2004). Engineering plant alkaloid biosynthetic pathways - progress and prospects. In Advances in Plant Biochemistry and Molecular Biology, Volume 1. W.D. Nes, and N.G. Lewis eds. Bioengineering and Molecular Biology of Plant Pathways. H.J. Bohnert, and Nguyen, H.T. eds (Elsevier Science Ltd. Oxford) in press.
Laemmli, U.K. (1970). Cleavage of structural proteins during the assembly of the head of bacteriophage T4. Nature 227, 680-685.
Lavid, N., Wang, J., Shalit, M., Guterman, I., Bar, E., Beuerle, T., Menda, N., Shafir, S., Zamir, D., Adam, Z., Vainstein, A., Weiss, D., Pichersky, E., and Lewinsohn, E. (2002). O-methyltransferases involved in the biosynthesis of volatile phenolic derivatives in rose petals. Plant Physiol. 129, 1899-18907.
Lenz, R., and Zenk, M.H. (1995a). Acetyl coenzyme A:salutaridinol-7-O-acteyltransferase from Papaver somniferum plant cell cultures. J. Biol. Chem. 270, 31091-31096.
Lenz, R., and Zenk, M.H. (1995b). Purification and properties of codeinone reductase (NADPH) from Papaver somniferum cell cultures and differentiated plants. Eur. J. Biochem. 233, 132-139.
Millgate, A.G., Pogson, B.J., Wilson, I.W., Kutchan, T.M., Zenk, M.H., Gerlach, W.L., Fist, A.J., and Larkin. P.J. (2004) Morphine-pathway block in top1 poppies. Nature 431, 413-414.
Morishige, T., Tsujita, T., Yamada, Y., and Sato, F. (2000). Molecular characterization of the S-adenosyl-L-methionine: 3'-hydroxy-N-methylcoclaurine 4'-O-methyltransferase involved in isoquinoline alkaloid biosynthesis in Coptis japonica. J. Biol. Chem. 275, 23398-23405.
Morishige, T., Dubouzet, E., Choi, K.B., Yazaki, K., and Sato, F. (2002). Molecular cloning of columbamine-O-methyltransferase from cultured Coptis japonica cells. Eur. J. Biochem. 269, 5659-5667.
Nakajima, K., Hashimoto, T., and Yamada, Y. (1993). Two tropinone reductases with different stereospecificities are short-chain dehydrogenases evolved from a common ancestor. Proc. Natl. Acad. Sci. USA 90, 9591-9595.
Nakajima, K., Oshita, Y., Kaya, M., Yamada, Y., and Hashimoto, T. (1999). Structures and expression patterns of two tropinone reductase genes from Hyoscyamus niger. Biosci. Biotechnol. Biochem. 63,1756-1764.
Nessler, C.L., and von der Haar, R.A. (1990). Cloning and expression analysis of DNA sequences fort he major latex protein of opium poppy. Planta 180, 487-491.
Ounaroon, A., Decker, G., Schmidt, J., Lottspeich, F., and Kutchan, T.M. (2003). (R,S)-Reticuline 7-O-methyltransferase and (R,S)-norcoclaurine 6-O-methyltransferase of Papaver somniferum - cDNA cloning and characterization of methyl transfer enzymes of alkaloid biosynthesis in opium poppy. Plant J. 36, 808-819.
Persson, B., Kallberg, Y., Oppermann, U., and Jörnvall, H. (2003). Coenzyme-based functional assignments of short-chain dehydrogenases/reductases (SDRs). Chem. Biol. Interact. 143-144, 271-278.
Preininger, V. (1986). Chemotaxonomy of Papaveraceae and Fumariaceae. In The Alkaloids, Vol. 29, A. Brossi, ed (San Diego: Academic Press), pp. 1-98.
Raith, K., Neubert, R., Poeaknapo, C., Boettcher, C., Zenk, M.H., and Schmidt, J. (2003). Electrospray tandem mass spectrometric investigations of morphinans. J. Am. Soc. Mass Spectrom. 14, 1262-1269.
Ringer, K.L., McConkey, M.E., Davis, E.M., Rushing, G.W., and Croteau, R. (2003). Monoterpene double-bond reductases of the (-)-menthol biosynthetic pathway: isolation and characterization of cDNAs encoding (-)-isopiperitenone reductase and (+)-pulegone reductase of peppermint. Arch. Biochem. Biophys. 418, 80-92.
Samanani, N., Liscombe, D.K., and Facchini, P.J. (2004). Molecular cloning and characterization of norcoclaurine synthase, an enzyme catalyzing the first committed step in benzylisoquinoline alkaloid biosynthesis. Plant J. 40, 302-313.
Sreenivasulu, N., Altschmied, L., Radchuk, V., Gubatz, S., Wobus, U., and Weschke, W. (2004). Transcript profiles and deduced changes of metabolic pathways in maternal and filial tissues of developing barley grains. Plant J. 37, 539-553.
Takeshita, N., Fujiwara, H., Mimura, H., Fitchen, J., Yamada, Y., and Sato, F. (1995). Molecular cloning and characterization of S-adenosyl-L-methionine: scoulerine 9-O-methyltransferase from cultured cells of Coptis japonica. Plant Cell Physiol 36, 29-36.
Thompson, J.D., Higgins, D.G., and Gibson, T.J. (1994). CLUSTAL W: improving the sensitivity of progressive multiple sequence alignment through sequence weighting, position-specific gap penalties and weight matrix choice. Nucleic Acids Res. 22, 4673-4680.
Unterlinner, B., Lenz, R., and Kutchan, T.M. (1999). Molecular cloning and functional expression of codeinone reductase: the penultimate enzyme in morphine biosynthesis in the opium poppy Papaver somniferum. Plant J. 18, 465-475.
Welle, R., Schröder, G., Schiltz, E., Grisebach, H., Schröder, J. (1991). Induced plant responses to pathogen attack. Analysis and heterologous expression of the key enzyme in the biosynthesis of phytoalexins in soybean (Glycine max L. Merr. cv. Harosoy 63). Eur. J. Biochem. 196, 423-430.
Xia, Z.Q., Costa, M.A., Pélissier, H.C., Davin, L.B., and Lewis, N.G. (2001). Secoisolariciresinol dehydrogenase purification, cloning, and functional expression. Implications for human health protection. J. Biol. Chem. 276, 12614-12623.
Ziegler, J., Díaz-Chávez, M.L., Kramell, R., Ammer, C., and Kutchan, T.M. (2005). Comparitive macroarray analysis of morphine containing Papaver somniferum and eight morphine free Papaver species identifies an O-methyltransferase involved in benzylisoquinoline biosynthesis. Planta 222, 458-471.

## Claims

1. A nucleic acid comprising a nucleotide sequence selected from the group consisting of:
(a) the nucleotide sequence as set forth in SEQ ID NO:1;
(b) a nucleotide sequence encoding the polypeptide as set forth in SEQ ID NO:2;
(c) a nucleotide sequence encoding a polypeptide having the amino acid sequence as set forth in SEQ ID NO:2 further comprising an amino-terminal methionine;
(d) a nucleotide sequence that is at least 70% identical to the nucleotide sequence of any of (a)-(c) over its entire length, wherein the nucleotide sequence encodes a polypeptide having salutaridine reductase activity;
(e) a nucleotide sequence encoding a polypeptide that is at least 70% identical to the polypeptide as set forth in SEQ ID NO:2 over its entire length, wherein the encoded polypeptide has salutaridine reductase activity;
(f) a nucleotide sequence which hybridizes under highly stringent or moderately stringent conditions to the nucleotide sequence as set forth in SEQ ID NO:1, wherein the nucleotide sequence encodes a polypeptide having salutaridine reductase activity;
(g) a nucleotide sequence that is an ortholog of the nucleotide sequence as set forth in SEQ ID NO:1, wherein the nucleotide sequence encodes a polypeptide having salutaridine reductase activity;
(h) a nucleotide sequence that is an allelic variant or a splice variant of the nucleotide sequence as set forth in SEQ ID N0:1, wherein the nucleotide sequence encodes a polypeptide having salutaridine reductase activity;
(i) a fragment of the nucleotide sequence of any of (a)-(h) encoding a polypeptide of at least 25 amino acid residues, wherein the encoded polypeptide has salutaridine reductase activity;
(j) a nucleotide sequence encoding a polypeptide as set forth in SEQ ID NO: 2 with at least one amino acid substitution, wherein the encoded polypeptide has salutaridine reductase activity;
(k) a nucleotide sequence encoding a polypeptide as set forth in SEQ ID NO:2 with at least one amino acid insertion, wherein the encoded polypeptide has salutaridine reductase activity;
(l) a nucleotide sequence encoding a polypeptide as set forth in SEQ ID NO:2 with at least one amino acid deletion, wherein the encoded polypeptide has salutaridine reductase activity;
(m) a nucleotide sequence encoding a polypeptide as set forth in SEQ ID NO:2 which has a carboxyl- and/or amino-terminal truncation, wherein the encoded polypeptide has salutaridine reductase activity;
(n) a nucleotide sequence encoding a polypeptide as set forth in SEQ ID NO:2 with at least one modification selected from the group consisting of amino acid substitution, amino acid insertion, amino acid deletion, carboxyl-terminal truncation, and amino-terminal truncation, wherein the encoded polypeptide has salutaridine reductase activity; and
(o) a nucleotide sequence complementary to any of (a)-(n), wherein the nucleotide sequence is at least 15 nucleotides in length.

2. An expression vector comprising the nucleic acid molecule of claim 1, operably linked to transcription regulatory sequences.

3. The expression vector of claim 2, wherein the transcription regulatory sequences are heterologous transcription regulatory sequences which are different from the native transcription regulatory sequences for salutaridine reductase.

4. A host cell comprising the expression vector of claim 2 or 3.

5. The host cell of claim 4, selected from a prokaryotic cell and a eukaryotic cell, preferably a bacterial cells, a yeast cell, a mammalian cells, and a plant cell.

6. A transgenic plant comprising the nucleic acid of claim 1 or the vector of claim 2 or 3, preferably the nucleic acid or the vector is stably integrated into the plant genome.

7. The transgenic plant of claim 6, wherein the plant belongs to the genus *Papaver.*

8. The transgenic plant of claim 7, wherein the plant belongs to a species selected from the group consisting of *Papaver somniferum, Papaver bracteatum, Papaver cylindricum, Papaver orientale, Papaver setigerum, Papaver pseudo-orientale, Papaver lauricola, Papaver persicum, Papaver caucasium, Papaver carmeli, Papaver arenarium, Papaver argemone, Papaver atlanticum, Papaver commutatum, Papaver dubium, Papaver fugax, Papaver hybridum, Papaver lateritium, Papaver oreophilum, Papaver pavonium, Papaver pilosum, Papaver pyrenaicum.*

9. Seed of the transgenic plant of any of claims 6-8.

10. Straw of the transgenic plant of any of claims 6-8.

11. A polypeptide encoded by a nucleic acid of any of claims 1 (a)-(n).

12. A fusion polypeptide comprising the polypeptide of claim 11 fused to a heterologous amino acid sequence.

13. An antibody or a fragment thereof that specifically binds to the polypeptide of claim 11.

14. The antibody or the fragment thereof of claim 13 that is a monoclonal antibody.

15. A hybridoma that produces the monoclonal antibody of claim 14.

16. A method for detecting and/or quantitating the amount of the polypeptide of claim 11, comprising the steps of:
(a) contacting a sample with the antibody or a fragment thereof of claim 13 or 14;
(b) detecting the complex formed between the polypeptide and the antibody or a fragment thereof; and
(c) optionally quantitating the amount of the complex.

17. A process for producing the polypeptide of claim 11 comprising culturing the host cell of claim 4 under suitable conditions to express the polypeptide, and optionally isolating the polypeptide from the culture, wherein the expression vector contained in the host cell of claim 4 comprises a nucleic acid of any of Claims 1 (a)-(n).

18. A method for producing a plant or a plant part having altered alkaloid production, comprising the steps of:
(a) introducing the nucleic acid of claim 1 into a cell or a part of an alkaloid-producing plant;
(b) growing the product of (a) under suitable conditions which permit the expression of the nucleic acid; and
(c) optionally regenerating a whole plant from said cell or part.

19. A method for producing a plant cell culture having altered alkaloid production, comprising the steps of:
(a) introducing the nucleic acid of claim 1 into a cell of an alkaloid-producing plant;
(b) growing the product of (a) under suitable conditions which permit the expression of the nucleic acid; and
(c) establishing a cell culture from said cell.

20. The method of claim 18 or 19, wherein the plant belongs to the genus *Papaver.*

21. The method of claim 20, wherein the plant belongs to a species selected from the group consisting of *Papaver somniferum, Papaver bracteatum, Papaver cylindricum, Papaver orientale, Papaver setigerum, Papaver pseudo-orientale, Papaver lauricola, Papaver persicum, Papaver caucasium, Papaver carmeli, Papaver arenarium, Papaver argemone, Papaver atlanticum, Papaver commutatum, Papaver dubium, Papaver fugax, Papaver hybridum, Papaver lateritium, Papaver oreophilum, Papaver pavonium, Papaver pilosum, Papaver pyrenaicum.*

22. A method for producing morphinan alkaloids, comprising the steps of:
(a) growing the plant obtainable by the method of claim 18 under suitable conditions which permit the expression of the nucleic acid of any of claims 1 (a)-(n), wherein the plant comprises the nucleic acid of any of claims 1 (a)-(n); and
(b) recovering morphinan alkaloids from the plant or the plant part.

23. A method of producing morphinan alkaloids, comprising the steps of:
(a) culturing the plant cell culture obtainable by the method of claim 19 under suitable conditions which permit the expression of the nucleic acid of any of claims 1(a)-(n), wherein the plant cell comprises the nucleic acid of any of claims 1(a)-(n); and
(b) recovering morphinan alkaloids from the plant cell culture.

24. A method of producing morphinan alkaloids, comprising the steps of:
(a) contacting a substrate-containing sample with the polypeptide of claim 11 under suitable conditions which allow the reduction of salutaridinee into salutaridinol; and
(b) recovering morphinan alkaloids.

25. The method of any of claims 22-24, wherein the morphinan alkaloid is salutaridinol.

26. Use of the nucleic acid of claim 1 or the vector of claim 2 or 3 for modulating alkaloid production in a plant, a plant part, or a plant cell.

27. Use of the host cell of claim 4, the transgenic plant of claim 6, the straw of claim 10, or the polypeptide of claim 11 for producing morphinan alkaloids.

28. Use of the transgenic plant of claim 6 or the straw of claim 10 for the preparation of a composition for reducing and/or eliminating pain and/or reducing and/or suppressing coughing.
